(19) **Europäisches Patentamt / European Patent Office / Office européen des brevets**

(11) **EP 2 643 339 B1**

(12) **FASCICULE DE BREVET EUROPEEN**

(45) Date de publication et mention
de la délivrance du brevet:
**07.01.2015 Bulletin 2015/02**

(21) Numéro de dépôt: **11794815.8**

(22) Date de dépôt: **14.11.2011**

(51) Int Cl.:
***C07H 15/04*** *(2006.01)*      ***C08G 65/00*** *(2006.01)*

(86) Numéro de dépôt international:
**PCT/FR2011/052635**

(87) Numéro de publication internationale:
**WO 2012/069730 (31.05.2012 Gazette 2012/22)**

(54) **NOUVEL AGENT HYDROTROPE, SON UTILISATION POUR SOLUBILISER DES TENSIOACTIFS NON-IONIQUES, COMPOSITIONS LES COMPRENANT**

NEUES HYDROTROPES MITTEL, SEINE VERWENDUNG ZUR LÖSLICHKEITSMACHUNG NICHT-IONISCHER TENSIDE UND ZUSAMMENSETZUNGEN DAMIT

NOVEL HYDROTROPIC AGENT, USE THEREOF TO MAKE NON-IONIC SURFACTANTS SOLUBLE, AND COMPOSITIONS CONTAINING SAME

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **25.11.2010  FR 1059762**

(43) Date de publication de la demande:
**02.10.2013  Bulletin 2013/40**

(73) Titulaire: **Societe D'Exploitation De Produits Pour Les**
**Industries Chimiques Seppic**
**75007 Paris (FR)**

(72) Inventeurs:
• **GAYRAL CHIRAC, Marie-Françoise**
  **F-81290 Viviers-les-Montagnes (FR)**
• **KERVERDO, Sébastien**
  **F-94300 Vincennes (FR)**
• **GUILBOT, Jerôme**
  **F-81100 Castres (FR)**
• **ROLLAND, Hervé**
  **F-81100 Castres (FR)**

(74) Mandataire: **Conan, Philippe Claude**
  **L'Air Liquide SA**
  **Direction de la Propriété Intellectuelle**
  **75, quai d'Orsay**
  **75321 Paris Cedex 07 (FR)**

(56) Documents cités:
WO-A1-91/03538      WO-A1-91/14760
WO-A1-96/33255      WO-A1-99/21948
WO-A1-2005/085321   FR-A1- 2 111 966
FR-A1- 2 754 739

• ADASCH, VOLKER ET AL: "Preparation of alkyl .alpha.- and .beta.-D-glucopyranosides, thermotropic properties and X-ray analysis", CARBOHYDRATE RESEARCH , 314(3-4), 177 -187 CODEN: CRBRAT; ISSN: 0008-6215, 1998, XP002622585,
• BERGER, BRYAN W. ET AL: "Relating surfactant properties to activity and solubilization of the human adenosine A3 receptor", BIOPHYSICAL JOURNAL , 89(1), 452-464 CODEN: BIOJAU; ISSN: 0006-3495, 2005, XP002622586,
• DAHLHOFF, WILHELM VOLKER: "Amphiphilic carbohydrate-based mesogens; I. Mesogenic O-n-alkyl .beta.-D-mannofuranosides: synthesis of a novel homologous series of glycosides", 1987, SYNTHESIS , (4), 366-8 CODEN: SYNTBF; ISSN: 0039-7881, XP002622587, Tableau 1 entrée 1
• DE BRUYNE, C. K. ET AL: "Acid hydrolysis of alkyl .beta.-D-galactopyranosides", CARBOHYDRATE RESEARCH , 25(1), 59-65 CODEN: CRBRAT; ISSN: 0008-6215, 1972, XP002622588,

**Description**

La présente invention concerne un nouvel agent tensioactif, son utilisation comme agent hydrotrope, notamment pour solubiliser des tensioactifs actifs non-ioniques, peu moussant dans des compositions aqueuses stables en milieu alcalin concentré, notamment mises en oeuvre pour le nettoyage.

Les agents hydrotropes sont des substances chimiques qui sont utilisés pour solubiliser des composés chimiques faiblement solubles ou insolubles dans de l'eau ou dans des phases aqueuses de compositions les comprenant. L'expression « composés chimiques faiblement solubles ou insolubles dans de l'eau ou dans des phases aqueuses » désigne des composés qui, ajoutés à une phase majoritairement ou totalement constituée d'eau ne permettent pas d'obtenir une solution ou une composition totalement limpide, transparente, isotrope, homogène et stable à une température souhaitée pendant une durée souhaitée. Ce défaut de solubilité est notamment du à la structure chimique du composé concerné et/ou à la présence d'agents alcalins et/ou d'électrolytes et/ou de sels neutres dans la phase aqueuse dans laquelle on souhaite solubiliser ledit composé.

Parmi les composés faiblement solubles ou insolubles dans l'eau, on peut citer des composés hydrophobes comme par exemple des huiles, des huiles essentielles, des fragrances, des pigments, des tensioactifs anioniques, des tensioactifs cationiques, des tensioactifs non ioniques et des tensioactifs amphotères.

Les agents hydrotropes sont particulièrement utiliser pour préparer des compositions cosmétiques, des compositions pharmaceutiques ; des compositions nettoyantes destinées à la détergence de surfaces dures pour des applications ménagères ou industrielles ou des compositions utilisées pour des opérations pétrolières renfermant des saumures.

Les compositions cosmétiques nécessitant l'utilisation d'agents hydrotropes sont généralement des compositions dans lesquelles le formulateur souhaite solubiliser des huiles et/ou des huiles essentielles et/ou des fragrances et/ou des agents tensioactifs en présence d'une quantité importante de sels neutres et/ou d'électrolytes.

Les compositions nettoyantes destinées à la détergence de surfaces dures pour des applications ménagères ou industrielles, telles que par exemple le nettoyage des bouteilles, le lavage des sols ou le nettoyage de surfaces métalliques souillées par des graisses, comprennent des agents tensioactifs détergents et également une forte concentration d'agents alcalins de façon à augmenter les performances nettoyantes desdites compositions. Ces compositions doivent ne pas générer la formation d'une mousse importante lors de l'opération de nettoyage en présence de la salissure à traiter, montrer de bonnes propriétés mouillantes et également bon pouvoir détergent en milieu alcalin.

Du fait de leur structure amphiphile, les agents tensioactifs détergents utilisés dans les compositions nettoyantes destinées à la détergence de surfaces dures pour des applications ménagères ou industrielles, confèrent à celles-ci sa capacité à éliminer les salissures présentes sur les surfaces dures et à les maintenir en suspension, pour être ensuite éliminées lors de l'étape de rinçage. Ces agents tensioactifs détergents peuvent être de nature anionique, cationique, amphotère ou non-ionique. Les tensioactifs non-ioniques sont particulièrement utilisés pour la préparation de compositions détergentes de surfaces dures compte tenu de leur pouvoir moussant généralement inférieur aux autres tensioactifs ioniques et également à leurs caractéristiques environnementales améliorées. Comme ces compositions nettoyantes comprennent de grandes quantités d'électrolytes, apportées par les agents alcalins et par des agents anticalcaires, comme par exemple les agents séquestrant et/ou les agents échangeurs d'ions et/ou les agents précipitant, il est difficile de dissoudre de grandes quantités de tensioactifs détergents pour obtenir une composition stable, ne présentant pas de dépôts au stockage.

Pour améliorer la solubilité de composés chimiques faiblement solubles ou insolubles dans l'eau ou dans des phases aqueuses, il est connu de l'homme du métier d'utiliser des agents hydrotropes comme par exemple des solvants organiques tels que l'éthanol, les xylènes sulfonates et les cumènes sulfonates. L'éthanol est un agent hydrotrope efficace mais il présente comme inconvénient de procurer des dangers d'explosivité. Les xylènes sulfonates et les cumènes sulfonates sont peu efficaces pour des grandes quantités de tensioactifs et ne présentent pas également des propriétés de biodégradabilités requises pour être en conformité avec les nouvelles réglementations environnementales.

Les alkylpolyglycosides sont également décrits comme agents solubilisant de tensioactifs non-ioniques démoussants.

La publication internationale WO96/33255A1 décrit des compositions anti-mousses comprenant un alkylpolyglucoside particulier, dont la chaîne alkyle est constituée par le radical 2-éthylhexyle et des agents tensioactifs non-ioniques démoussants choisis parmi ceux comprenant un ou plusieurs groupes choisis parmi les groupes mono-éthoxylés ou poly-éthoxylés, les groupes mono-propoxylés ou poly-propoxylés. Il y est enseigné que les alkylpolyglucosides à chaîne 2-éthylhexyle sont plus efficaces que les alkyl polygylcosides à chaine hexyle pour solubiliser des tensioactifs non-ioniques démoussants.

La publication internationale WO99/21948A1 divulgue des compositions limpides et stables à hautes concentrations alcalines, dont les propriétés moussantes sont contrôlées, contenant une grande quantité de tensioactifs non-ioniques à base d'oxyde d'alkylène et un hexylglycoside comme agent hydrotrope. Ces compositions se caractérisent par un bon pouvoir mouillant et de bonnes propriétés détergentes de surfaces dures. Il y est enseigné que les hexylglycosides et plus particulièrement le n-hexylpolyglucoside permettent de solubiliser dans des milieux fortement alcalins des tensioactifs non-ioniques et que le n-hexylglucoside se caractérise par un pouvoir solubilisant supérieur au 2-éthylhexylglucoside et à l'Exxal 7 glucoside en présence de quantités de soude comprises entre 10% et 40%, pour un tensioactif non-ionique dont la structure résulte de l'éthoxylation par 4 moles d'oxyde d'éthylène d'un mélange d'alcools linéaires et ramifiés, avec un taux d'alcools linéaires d'environ 80%, comprenant de 9 à 11 atomes de carbone. Cependant, ni le 2-éthylhexylglucoside, ni le n-hexylglucoside ne se caractérisent par des propriétés permettant de solubiliser à la fois une grande quantité d'agent alcalin et une grande quantité d'électrolytes apportées par les agents anticalcaires.

La demanderesse s'est donc attachée à développer une solution technique nouvelle, consistant en un nouveau agent hydrotrope, qui permet plus particulièrement de préparer des compositions comprenant des tensioactifs détergents non-ioniques et présente des caractéristiques non écotoxiques et biodégradables et non inflammable, permettant de solubiliser des composés faiblement solubles ou insolubles dans l'eau, et plus particulièrement de solubiliser des tensioactifs non-ioniques dans un milieu aqueux en présence d'une forte teneur en agent alcalin et/ou en électrolytes et/ou en seuls neutres. C'est pourquoi, selon un premier aspect, l'invention a pour objet une composition de formule (I) :

$$nC_7H_{15}\text{-}O\text{-}(G)_p\text{-}H \qquad (I)$$

dans laquelle G représente le reste d'un sucre réducteur, et p représente un nombre décimal supérieur ou égal à 1,05 et inférieur ou égal à 5, ladite formule (I) représentant un mélange de composés :

$$a_1\, nC_7H_{15}\text{-}O\text{-}(G)_1\text{-}H + a_2 nC_7H_{15}\text{-}O\text{-}(G)_2\text{-}H + a_3 nC_7H_{15}\text{-}O\text{-}(G)_3\text{-}H + ... + a_q\, nC_7H_{15}\text{-}O\text{-}(G)_q\text{-}H$$

avec q représentant un nombre entier compris entre 1 et 5 et dans les proportions molaires $a_1, a_2, a_3,... a_q$ telles que :

$$\sum_{q=5}^{q=1} a_q = 1 \;;\; a_1 > 0\,.$$

Dans la définition de la formule (I) telle que définie précédemment, p est un nombre décimal qui représente le degré moyen de polymérisation du reste G.

Par sucre réducteur, on désigne dans la formule (I), les dérivés saccharidiques qui ne présentent pas dans leurs structures de liaison glycosidique établie entre un carbone anomérique et l'oxygène d'un groupement acétal tels qu'ils sont définis dans l'ouvrage de référence : « Biochemistry », Daniel Voet/Judith G. Voet, p. 250, John Wyley & Sons, 1990. La structure oligomérique $(G)_p$, peut se présenter sous toutes formes d'isoméries, qu'il s'agisse d'isomérie optique, d'isomérie géométrique ou d'isomérie de position ; elle peut aussi représenter un mélange d'isomères.

Dans la formule (I) telle que définie ci-dessus, le groupe $nC_7H_{15}$-O est lié à G par le carbone anomérique du reste saccharide, de manière à former une fonction acétal.

Selon un aspect particulier de la présente invention, dans la formule (I), G représente le reste d'un sucre réducteur choisi parmi le glucose, le dextrose, le saccharose, le fructose, l'idose, le gulose, le galactose, le maltose, l'isomaltose, le maltotriose, le lactose, le cellobiose, le mannose, le ribose, le xylose, l'arabinose, le lyxose, l'allose, l'altrose, le dextrane ou le tallose.

Selon un aspect particulier de la présente invention, dans la formule (I), G représente le reste d'un sucre réducteur choisi parmi les restes du glucose, du xylose et de l'arabinose, et p représente un nombre décimal supérieur ou égal à 1,05 et inférieur ou égal à 2,5.

Selon un aspect encore plus particulier de la présente invention, dans la formule (I), G représente le reste d'un sucre réducteur est choisi parmi les restes du glucose, du xylose et de l'arabinose, et p représente un nombre décimal supérieur ou égal à 1,05 et inférieur ou égal à 2,0, et encore plus particulièrement supérieur ou égal à 1,25 et inférieur ou égal à 2,0.

Selon un deuxième aspect, l'invention a pour objet un procédé de préparation de la composition de formule (I) telle que définie précédemment, comprenant les étapes successives suivantes :

-    une <u>étape A)</u> de réaction d'un sucre réducteur de formule (III) :

$$HO\text{-}(G)p\text{-}H \ (III)$$

dans laquelle G représente le reste d'un sucre réducteur, avec un excès molaire de n-heptanol de formule $nC_7H_{15}$-OH, pour former un mélange de composés de formule (I) telle que définie précédemment et de n-heptanol ;
-    une <u>étape B)</u> d'élimination du n-heptanol dudit mélange obtenu à <u>l'étape A).</u>

L'étape A) est généralement mise en oeuvre dans un réacteur en présence d'un système catalytique acide, en maîtrisant le rapport stoechiométrique entre les deux réactants, et plus particulièrement en introduisant un excès molaire de n-heptanol, et sous agitation mécanique dans des conditions de température et de vide partiel prédéterminées, par exemple à une température comprise entre 70°C et 130°C et sous un vide partiel compris entre 300 mbar ($3.10^4$ Pa) et 20 mbar ($2.10^3$ Pa). Par système catalytique acide, on désigne les acides forts comme l'acide sulfurique, l'acide chlorhydrique, l'acide phosphorique, l'acide nitrique, l'acide hypophosphoreux, l'acide méthanesulfonique, l'acide (paratoluène) sulfonique, l'acide (trifluorométhane) sulfonique, ou les résines échangeuses d'ions.

L'étape B) d'élimination du n-heptanol dudit mélange obtenu à l'issue de l'étape A) est généralement mise en oeuvre selon des méthodes connues de l'homme du métier comme par exemple, la distillation, la distillation sur film à couche mince, la distillation moléculaire ou l'extraction par solvants.

Un tel procédé de préparation peut être complété, si nécessaire ou si désiré, par des opérations de neutralisation, de filtration et de décoloration.

Selon un autre aspect, l'invention a pour objet l'utilisation d'une composition de formule (I) telle que précédemment, comme agent tensioactif hydrotrope.

L'expression « agent tensioactif hydrotrope » désigne un agent tensioactif permettant de solubiliser des composés faiblement solubles ou insolubles dans la phase aqueuse d'une composition, par mélange dudit agent tensioactif hydrotrope et du composé à solubiliser dans la phase aqueuse d'une composition.

L'expression « composés faiblement solubles ou insolubles dans l'eau » désigne des composés qui, ajoutés à une phase majoritairement ou totalement constituée d'eau et en présence d'agents alcalins et/ou d'électrolytes et/ou de sels neutres, ne permettent pas d'obtenir une solution ou une composition limpide, transparente, isotrope, homogène et stable à la température souhaitée. Parmi ces composés faiblement solubles ou insolubles dans l'eau, on peut citer par exemple des huiles, des huiles essentielles, des fragrances, des pigments, des tensioactifs anioniques, des tensioactifs cationiques, des tensioactifs non-ioniques et des tensioactifs amphotères. Parmi les sels neutres, on peut citer le chlorure d'ammonium, les sels de métaux alcalins comme par exemple le chlorure de sodium, le sulfate de sodium, le nitrate de sodium, les sels de métaux alcalino-terreux comme par exemple le chlorure de calcium.

La composition de formule (I) utilisée comme agent tensioactif hydrotrope objet de la présente invention et telle que définie précédemment, peut être incorporée dans des compositions cosmétiques, dermocosmétiques, dermopharmaceutiques, pharmaceutiques ou de détergence industrielle ou ménagère.

Selon un autre aspect, l'invention a pour objet l'utilisation de la composition de formule (I) telle que définie précédemment, comme agent solubilisant, dans une composition alcaline aqueuse, d'au moins un tensioactif non-ionique de formule (II) :

$$R\text{-}(O\text{-}CH(R')\text{-}CH_2)_n\text{-}(O\text{-}CH_2\text{-}CH_2)_m\text{-}O\text{-}H \qquad (II)$$

dans laquelle R représente un radical aliphatique hydrocarboné, saturé ou insaturé, linéaire ou ramifié, comportant de 8 à 14 atomes de carbone, R' représente un radical méthyle ou éthyle, n représente un nombre entier supérieur ou égal à 0 et inférieur ou égal à 15, m représente un nombre entier supérieur ou égal à 0 et inférieur ou égal à 15, étant entendu que la somme n + m est supérieure à zéro.

L'expression « composition alcaline aqueuse » désigne toute composition aqueuse qui présente une valeur de pH supérieure à 7.

Par radical aliphatique hydrocarboné, saturé ou insaturé, linéaire ou ramifié, comportant de 8 à 14 atomes de carbone, éventuellement substitué avec un ou plusieurs groupes hydroxyle, on désigne pour le radical R dans la formule (II) telle que définie ci-dessus :

- Les radicaux alkyle linéaires, par exemple les radicaux n-octyle, n-décyle, n-dodécyle ou n-tétradécyle ;
- Les radicaux issus des isoalcanols de formule (1) :

$$(CH_3)(CH_3)CH\text{-}(CH_2)_r\text{-}CH_2\text{-}OH \qquad (1)$$

dans laquelle r représente un nombre entier compris entre 4 et 10, par exemple les radicaux isooctyle, isononyle, isodécyle, isoundécyle, isododécyle, isotridécyle ou isotétradécyle ;
- Le radical 2-éthyl hexyle ou les radicaux alkyles ramifiés issus des alcools de Guerbet de formule (2) :

$$CH(C_sH_{2s+1})(C_tH_{2t+1})\text{-}CH_2\text{-}OH \qquad (2)$$

dans laquelle t est un nombre entier compris entre 4 et 10, s est un nombre entier compris entre 2 et 10 et la somme s + t est supérieure ou égale à 6, et inférieure ou égale à 12, par exemple les radicaux 2-éthyl décyle, 2-butyl octyle, 2-éthyl dodécyle, 2-butyl décyle, 2-hexyl octyle, 2-butyl décyle, 2-hexyl octyle ; ou les radicaux issus des homologues d'alcools de Guerbet, par exemple le radical 2-propyl heptyle.

- Les radicaux issus des alcools ramifiés de formule (3) :

$$CH_3\text{-}[CH(R'')]_z\text{-}CH_2\text{-}OH \qquad (3)$$

dans laquelle R'' représente un atome d'hydrogène ou un radical méthyle, et z représente un nombre entier supérieur ou égal à 3 et inférieur ou égal à 15.
- Les radicaux linéaires insaturés tels que les radicaux undécènyle, dodécènyle ou tétradécènyle, comme par exemple les radicaux insaturés 10-undécènyle, 4-dodécènyle, ou 5-dodécènyle ;
- Les radicaux aliphatiques saturés ou insaturés, linéaires ou ramifiés, comportant de 8 à 14 atomes de carbone substitués par un ou deux groupes hydroxy, tels que les radicaux hydroxy octyle, hydroxydécyle, hydroxydodécyle, par exemple les radicaux 8-hydroxy octyle, 10-hydroxy décyle ou 12-hydroxy dodécyle.

Selon un aspect particulier de la présente invention, celle-ci a pour objet l'utilisation d'une composition de formule (I) pour solubiliser dans une composition alcaline au moins un tensioactif non-ionique de formule (II) telle que définie précédemment dans laquelle le radical R représente un radical choisi parmi les radicaux octyle, décyle, dodécyle, tétradécyle, 2-éthyl hexyle, 2-butyl octyle, 2-butyl décyle, 2-hexyl octyle, isooctyle, isononyle, isodécyle, isoundécyle, isododécyle, isotridécyle, isotétradécyle ou 2-propyl heptyle.

Selon un aspect particulier de la présente invention, celle-ci a pour objet l'utilisation d'une composition de formule (I) pour solubiliser dans une composition alcaline au moins un tensioactif non-ionique de formule (II) telle que définie précédemment dans laquelle n représente un nombre entier supérieur ou égal 0 et inférieur ou égal à 6, plus particulièrement supérieur ou égal à 0 et inférieur ou égal à 3, et encore plus particulièrement supérieur ou égal à 0 et inférieur ou égal à 2.

Selon un aspect plus particulier de la présente invention, celle-ci a pour objet l'utilisation d'une composition de formule (I) pour solubiliser dans une composition alcaline au moins un tensioactif non-ionique de formule (II) telle que définie précédemment dans laquelle m représente un nombre entier supérieur ou égal à 1 et inférieur ou égal à 9, plus particulièrement supérieur ou égal à 2 et inférieur ou égal à 4.

Les composés de formule (II) pour lesquels R' représente un radical méthyle ou éthyle et n représente un nombre entier supérieur ou égal à 1 sont préparés selon un procédé comprenant si nécessaire une étape a) d'alcoxylation par réaction de n équivalents molaire d'un oxyde d'alkylène ou d'un carbonate d'alkylène avec un équivalent molaire d'alcool de formule (IV) :

$$R\text{-}OH \qquad (IV)$$

dans laquelle le radical R représente un radical aliphatique hydrocarboné, saturé ou insaturé, linéaire ou ramifié, comportant de 8 à 14 atomes de carbone, éventuellement substitué avec un ou plusieurs groupes hydroxyle, tel que défini ci-dessus, pour obtenir l'alcool alcoxylé de formule (V):

$$R\text{-}(O\text{-}CH(R')\text{-}CH_2)_n\text{-}O\text{-}H \qquad (V)$$

dans laquelle R' représente un radical méthyle ou éthyle ; et/ou si nécessaire une étape b) d'éthoxylation par réaction d'un équivalent molaire de l'acool alcoxylé de formule (V) obtenu à l'issu de l'étape a) avec m équivalents molaire d'oxyde d'éthylène ou de carbonate d'éthylène.

Dans l'étape a) du procédé de préparation des composés de formule (II) telle que décrite ci-dessus, l'oxyde d'alkylène est choisi parmi les éléments du groupe constitué par l'oxyde de propylène et l'oxyde de butylène, et le carbonate d'alkylène est choisi parmi les éléments du groupe constitué par le carbonate de propylène et le carbonate de butylène.

Les composés de formule (II) pour lesquels n est égal à 0 sont préparés selon un procédé mettant en oeuvre une étape a') d'éthoxylation par réaction de m équivalent molaire d'oxyde d'éthylène ou de carbonate d'éthylène avec l'alcool de formule (IV) telle que définie ci-dessus.

Dans les procédés décrits ci-dessus, n et m représentent les nombres entiers décrits ci-dessus dans la définition des composés de la formule (II).

Les réactions d'alcoxylation de l'étape a) et d'éthoxylation des étapes a') et b), telles que définies ci-dessus, sont généralement mises en oeuvre dans un réacteur en présence d'un catalyseur basique tel que les hydroxydes de métaux alcalins, comme par exemple la soude, la potasse, les alcoolates de métaux alcalins, comme par exemple le méthylate de sodium ou de potassium, le tertiobutylate de sodium ou de potassium, les bases de Lewis comme par exemple la triphenylphosphine, les catalyseurs de coordination comme par exemple des complexes organométalliques à base de cobalt et/ou de zinc, ou en présence d'un catalyseur acide tel qu'un acide de Lewis comme par exemple le trifluorure de bore, le trichlorure d'aluminium ou le tétrachlorure d'étain.

De tels procédés de préparation des composés de formule (II) peuvent être complétés, si nécessaire ou si désiré, par des opérations de neutralisation, de déminéralisation, de filtration et de décoloration.

Selon un autre aspect particulier de la présente invention, celle-ci a pour objet l'utilisation d'une composition de formule (I) pour solubiliser au moins un tensioactif non-ionique de formule (II) telle que définie ci-dessus dans une composition alcaline aqueuse dans laquelle le rapport massique entre le composé de formule (I) et le composé de formule (II) est inférieur ou égal à 9/1 et supérieur ou égal à 1/4.

Selon un autre aspect plus particulier de la présente invention, celle-ci a pour objet l'utilisation d'une composition de formule (I) pour solubiliser au moins un tensioactif non-ionique de formule (II) telle que définie ci-dessus dans une composition alcaline aqueuse dans laquelle le rapport massique entre la composition de formule (I) et le composé de formule (II) est inférieur ou égal à 4/1 et supérieur égal à 1/4, plus particulièrement inférieur ou égal à 3/1 et supérieur ou égal à 1/3, encore plus particulièrement inférieur ou égal à 2/1 et supérieur ou égal à 1/2.

Selon un autre aspect, l'invention a pour objet une composition ($C_1$) comprenant pour 100% de sa masse :

a) de 0,5 % à 20 % massique, plus particulièrement de 0,5% à 15 % massique, et encore plus particulièrement de 0,5% à 10 % massique d'une composition de formule (I), telle que définie ci-dessus ;
b) de 0,5 % à 80 % massique, plus particulièrement de 0,5 % à 50 % massique, et encore plus particulièrement de 0,5 % à 35 % massique d'au moins un tensioactif non-ionique de formule (II) :

$$R\text{-}(O\text{-}CH(R')\text{-}CH_2)_n\text{-}(O\text{-}CH_2\text{-}CH_2)_m\text{-}O\text{-}H \qquad (II)$$

dans laquelle R représente un radical aliphatique hydrocarboné, saturé ou insaturé, linéaire ou ramifié, comportant de 8 à 14 atomes de carbone, R' représente un radical méthyle ou propyle, n représente un nombre entier supérieur ou égal à 0 et inférieur ou égal à 15 , m représente un nombre entier supérieur ou égal à 0 et inférieur ou égal à 15, étant entendu que la somme n + m est supérieure à zéro ;
c) de 10 % à 50 % massique, plus particulièrement de 20 % à 50 % massique et encore plus particulièrement de 20 % à 50 % massique d'au moins un agent alcalin choisi parmi les éléments du groupe constitué par les hydroxydes de métaux alcalins ou alcalino-terreux ;
d) de 15 % à 89 % massique, plus particullièrement de 15 % à 50 % massique, et encore plus particulièrement de 20 % à 50 % massique d'eau. ; et optionnellement
e) de 10 % à .50 % massique, plus particulièrement de 20 % à 50 % massique, et encore plus particulièrement de 30 % à 50 % massique d'au moins un agent anticalcaire.

Dans la composition ($C_1$) objet de la présente invention, les agents alcalins sont choisis parmi les éléments du groupe constitué par les hydroxydes de métaux alcalins ou alcalino-terreux comme par exemple l'hydroxyde de sodium, l'hydroxyde de potassium, l'hydroxyde de barium et l'hydroxyde de calcium.

Selon un aspect encore plus particulier, dans la composition ($C_1$) objet de la présente invention, l'agent alcalin est choisi parmi les éléments du groupe constitué de l'hydroxyde de sodium, l'hydroxyde de potassium.

Dans la composition ($C_1$) objet de la présente invention, l'agent anticalcaire optionnel est choisi parmi les éléments du groupe constitué par les agents séquestrants, comme par exemple le tripolyphospate de sodium (TPP), l'éthylènediaminetétracétate (EDTA), le tetraacetyléthylènediamine (TAED), le méthyl glycine diacétate (MGDA), le nitrolotriacétate de sodium ($Na_3NTA$), les gluconates de sodium ou de potassium, les érythorbates de sodium ou de potassium, les polycarboxylates de sodium ou de potassium, et le citrate de sodium, par les agents échangeurs d'ions comme par exemple les zéolithes ou aluminosilicates de sodium, ou les silicates de sodium lamellaires, les agents précipitants comme par exemple le carbonate de calcium et le métasilicate de sodium.

Les agents séquestrants, et plus particulièrement les agents séquestrants décrits ci-dessus, ont pour effet de complexer les ions calcium et magnésium pour former des complexes hydrosolubles ensuite éliminés pendant le rinçage.

Les agents échangeurs d'ions, et plus particulièrement les agents échangeurs d'ions décrits ci-dessus, ont pour effet d'échanger leurs ions sodium avec des ions calcium et magnésium.

Les agents précipitants, et plus particulièrement les agents séquestrants décrits ci-dessus, ont pour effet d'éliminer les ions responsables de la dureté de l'eau en formant des composés de calcium insolubles, éliminé par la suite avec les salissures sur les surfaces nettoyées.

Selon un aspect plus particulier, dans la composition ($C_1$) objet de la présente invention, l'agent anticalcaire optionnel est choisi parmi les éléments du groupe constitué du métasilicate de sodium, du tripolyphospate de sodium (TPP), de l'éthylènediaminetétracétate (EDTA), du tetraacetyléthylènediamine (TAED), du méthyl glycine diacétate (MGDA), du nitrolotriacétate de sodium ($Na_3NTA$), du gluconate de sodium, du citrate de sodium et du carbonate de calcium.

Selon un aspect particulier, dans la composition ($C_1$) objet de la présente invention, le rapport massique entre le composé de formule (II) et la composition de formule (I) est inférieur ou égal à 9/1 et supérieur égal à 1/4, plus particulièrement inférieur ou égal à 4/1 et supérieur ou égal à 1/4, plus particulièrement inférieur ou égal à 3/1 et supérieur ou égal à 1/3, encore plus particulièrement inférieur ou égal à 2/1 et supérieur ou égal à 1/2.

Selon un autre aspect, l'invention a pour objet l'utilisation d'une composition ($C_1$) telle que définie ci-dessus pour nettoyer les surfaces dures.

L'expression « pour nettoyer les surfaces dures » désigne toute action destinée à permettre l'élimination de salissures présentes sur des surfaces constituées de matériaux divers. Les surfaces à nettoyer peuvent être des surfaces dures ou des surfaces textiles. Par surfaces dures, on désigne par exemple les sols, les murs, les carreaux de fenêtres, les carrelages, les appareils électro-ménagers, la vaisselle, les plans de travail, la robinetterie, les éviers, des cuves de stockage de produits chimiques, alimentaires ou agricoles, les véhicules (automobiles, moto, camions, ...).

Les matériaux constituant ces surfaces dures sont par exemple le verre (sodocalcique, fluorocalcique, borosilicate, cristal), la porcelaine, la faïence, la céramique, les plastiques polycarbonates, polypropylènes, l'acier inoxydable, l'argent, le cuivre, l'aluminium, le bois, les résines synthétiques, la vitrocéramique, le linoleum, et peuvent être revêtus de peintures, de vernis.

Comme exemple de salissures présentes sur ces surfaces dures et à éliminer par nettoyage, on peut citer par exemple des résidus alimentaires, des graisses, des hydrocarbures lourds et légers, des résidus brûlés, de la poussière, de la boue, des traces de doigts, des résidus de savon, des germes.

La composition ($C_1$) objet de la présente invention se présente notamment sous la forme d'une solution, d'une émulsion ou d'une microémulsion à phase continue aqueuse, d'une émulsion ou d'une microémulsion à phase continue huileuse, d'un gel, d'une mousse, ou encore sous la forme d'un aérosol.

La composition ($C_1$) objet de la présente invention peut être appliquée directement par aspersion ou par vaporisation sur la surface à nettoyer ou bien par l'intermédiaire de tout type de support destiné à être mis en contact avec la surface dure à nettoyer (papier, lingette, textile) comprenant ladite composition ($C_1$).

La composition ($C_1$) objet de la présente invention, utilisée pour nettoyer des surfaces dures, présente généralement un pH supérieur à 9, de préférence supérieur à 11, et plus particulièrement supérieur à 13.

De façon générale la composition ($C_1$) objet de la présente invention, comporte également des ingrédients habituellement mis en oeuvre dans le domaine du nettoyage de surfaces dures, comme des tensioactifs non-ioniques, des agents tensioactifs cationiques, des polymères cationiques, des agents épaississants, des enzymes, des agents de blanchiment, des agents anticorrosion, des agents conservateurs, des parfums, des colorants, des agents répulsifs.

Comme exemples de tensioactifs non-ioniques présents dans la composition ($C_1$) objet de la présente invention, on peut citer :

- Des copolymères blocs d'oxyde d'éthylène et d'oxyde de propylène, et tout particulièrement les copolymères blocs d'oxyde d'éthylène et d'oxyde de propylène commercialisés sous le nom de marque PLURONIC™ par la société BASF, comme par exemple le PLURONIC™ PE 6100 et le PLURONIC™ PE 6200 ,
- Des tensioactifs non ioniques démoussant de formule ($A_1$) :

$$R_1\text{-}X\text{-}[(CH_2\text{-}CH(CH_3)\text{-}O)_U\text{-}(CH_2\text{-}CH_2\text{-}O)_V\text{-}Y]_W \qquad (A_1)$$

dans laquelle :

- $R_1$ représente un radical aliphatique hydrocarboné, saturé ou insaturé, linéaire ou ramifié, comportant de 6 à 18 atomes de carbone,
- X représente un atome d'azote ou un atome d'oxygène,
- v représente un nombre entier compris entre 1 et 50,
- u représente un nombre entier compris entre 1 et 50,
- w représente un nombre entier égal à 1 si X représente un atome d'oxygène, et w représente un nombre entier égal à 1 ou 2 si X représente un atome d'azote.
- Y représente un groupe fonctionnel bloquant choisi parmi les éléments du groupe constitué par les radicaux alkyles linéaires comportant de 4 à 8 atomes de carbone, comme par exemple le radical butyle, le radical benzyle, un groupe oxyde de butylène.

Parmi les tensioactifs non ioniques démoussants de formule ($A_1$), on peut citer les produits commercialisés sous le nom de marque TERGITOL™ par la société DOW CHEMICAL comme par exemple le TERGITOL™ L61 E et le TERGITOLT™ L64E

- Des tensioactifs non ioniques peu moussant de formule ($A_2$) :

$$R_2\text{-}O\text{-}(S)_q\text{-}H \qquad (A_2)$$

dans laquelle :

- S représente le reste d'un sucre réducteur choisis parmi les éléments du groupe constitué par le glucose, le xylose et l'arabinose,
- $R_2$ représente un radical hydrocarboné saturé, linéaire ou ramifié, comportant de 6 à 10 atomes de carbone
- q représente un nombre décimal supérieur ou égal à 1,05 et inférieur ou égal à 5.

Comme exemple de tensioactifs non ioniques peu moussants de formule ($A_2$) présents dans la composition ($C_1$) objet de la présente invention, on peut citer les hexylpolyglucosides et les 2-ethylpolyglucosides.

Comme exemples d'agents épaississants présents dans la composition ($C_1$) objet de la présente invention, on peut citer les agents épaississants polymériques comme les polycarboxylates possédant un poids moléculaire compris entre 500,000 et 4,000,000 grammes par mole, plus particulièrement entre 1,000,000 et 4,000,000 grammes par mole, avec un taux de réticulation compris entre 0,5% et 4% molaire, comme ceux commercialisés sous les noms de marque Carbopol™, Acrysol™ICS-1 et Sokalan™. Les polycarboxylates préférés sont des polyacrylates, ou des copolymères de l'acide acrylique avec l'éthylène, avec le propylène ou l'acide maléique.

Comme exemples d'agents épaississants présents dans la composition ($C_1$) objet de la présente invention, on peut citer les agents épaississants polymériques comme les homopolymères de l'acrylamide, ou les copolymères de l'acrylamide et du sel de sodium du 2-acrylamido-2-méthylpropanesulfonate, comme par exemple les épaississants commercialisés par la société SEPPIC sous le nom de marque de SOLAGUM™.

Comme exemples d'agents épaississants présents dans la composition ($C_1$) objet de la présente invention, on peut citer les agents épaississants inorganiques comme par exemple les argiles, la montmorillonite (ou bentonite), la volchonskoite, la smectite, la nontronite, beidellite, l'hectorite, la saponite, la sauconite ou la vermiculite.

Les agents épaississants présents dans la composition ($C_1$) objet de la présente invention sont utilisés dans des quantités comprises entre 0,1% et 10% massique.

Comme exemples d'agents abrasifs présents dans la composition ($C_1$) objet de la présente invention, on peut citer citer des matériaux d'origine naturelle comme par exemple des copeaux de bois ou de noyaux, des matériaux abrasifs inorganiques tels que des oxydes, des carbonates, des quartzs, des terres diatomées, des dioxydes de silice colloïdales, des matériaux abrasifs organiques tels que des polyoléfines comme les polyéthylènes et les polypropylènes, des polyesters, des polystyrènes, des résines d'acétonitrile-butadiène-styrène, des mélamines, des polycarbonates, des résines phénoliques, des résines époxy, des résines polyuréthanes.

Les agents abrasifs présents dans la composition ($C_1$) objet de la présente invention sont utilisés dans des quantités comprises entre 5,0% et 30% massique.

Comme exemples d'enzymes présentes dans la composition ($C_1$) objet de la présente invention, on peut citer les protéases, les lipases et les amylases.

Comme exemples d'agents de blanchiment présents dans la composition ($C_1$) objets de la présente invention, on peut citer l'hypochlorite de sodium, les composés peroxygénés comme le percarbonate de calcium, les perborates.

Selon un autre aspect, l'invention a pour objet un procédé de nettoyage d'une surface dure comprenant au moins une étape $a_1$) d'application de la composition ($C_1$) telle que définie ci-dessus sur ladite surface dure, suivie d'au moins une étape $b_1$) de rinçage de ladite surface dure.

Dans l'étape $a_1$) du procédé de nettoyage objet de l'invention, la composition ($C_1$) est appliquée sur la surface comprenant les salissures à nettoyer par tout moyen comme par exemple en plein bain, par aspersion, par application par l'intermédiaire d'un support constitué de_fibres textiles synthétiques ou naturelles, tissées ou non tissées, ou de papier, préalablement imprégné de ladite composition ($C_1$).

Dans l'étape $b_1$) du procédé de nettoyage objet de l'invention, le rinçage de la surface dure sur laquelle a été appliquée la composition ($C_1$) lors de l'étape $a_1$) est réalisé en plein bain ou par aspersion d'eau,.

L'étape $b_1$) du procédé de nettoyage objet de l'invention, peut être réalisée à température ambiante ou à une température comprise entre 30°C et 80°C, plus particulièrement à une température comprise entre 30°C et 65°C.

Les exemples suivants illustrent l'invention, sans toutefois la limiter.

## 1) Préparation d'une composition de formule (I) objet de l'invention et évaluation de ses propriétés tensioactives.

### 1.1) Préparation de n-heptylpolyglucosides

On introduit 2,7 équivalents molaires de n-heptanol dans un réacteur en verre à double enveloppe, dans laquelle circule un fluide caloporteur, et muni d'une agitation efficace, à une température de 40°C. Un équivalent molaire de glucose anhydre est alors ajouté progressivement au milieu réactionnel pour permettre sa dispersion homogène, puis 0,15 % massique d'acide sulfurique à 98% et 0,15 % massique d'acide hypophosphoreux à 50% pour 100% de la masse constituée par la somme de la massse du glucose et de la masse du n-heptanol sont introduits dans la dispersion homogène préalablement préparée. Le milieu réactionnel est placé sous un vide partiel d'environ 180 mbars, et maintenu à une température de 100°C-105°C pendant une durée de 4 heures avec évacuation de l'eau formée au moyen d'un montage de distillation. Le milieu réactionnel est ensuite refroidi à 85°C-90°C et neutralisé par ajout de soude à 40 %, pour amener le pH d'une solution à 5 % de ce mélange à une valeur d'environ 7,0. La milieu réactionnel ainsi obtenu est ensuite vidangée à une température de 70°C et filtré pour éliminer les grains de glucose n'ayant pas réagi. Le filtrat est ensuite introduit dans un réacteur en verre à double enveloppe, dans laquelle circule un fluide caloporteur, muni d'une agitation efficace et d'un dispositif de distillation. L'excès d'heptanol est ensuite éliminé par distillation à une température de 120°C sous vide partiel compris entre environ 100 mbars et 50 mbars. Le milieu réactionnel ainsi distillé est aussitôt dilué par l'ajout d'une quantité d'eau de façon à atteindre une concentration en milieu réactionnel d'environ 60%. Après homogénéisation pendant 30 minutes à une température de 50°C, la composition ($X_0$) obtenue est vidangée.

Les caractéristiques analytiques de la composition ($X_0$) ainsi obtenue comprenant des n-heptylpolyglucosides sont rassemblées dans le tableau 1 ci-dessous.

**Tableau 1 :** Caractéristiques analytiques de la composition ($X_0$)

|  | Composition ($X_0$) |
|---|---|
| Aspect à 20°C (Détermination visuelle) | Liquide |
| Indice d'acide (Norme NFT 60204) | 1,7 |
| Indice d'hydroxyle sur extrait sec (Norme USP XXI NF XVI 01/01/1995) | 813,9 |
| Eau (% massique) (Norme NFT 73201) | 58,8% |
| Teneur résiduelle en n-heptanol (chromatographie en phase gazeuse) en % massique | 0,22% |

**1.2) Evaluation des propriétés moussantes de n-heptylpolyglucosides**

Les propriétés moussantes de la composition ($X_0$) de n-heptylpolyglucosides, obtenue selon le procédé précédemment décrit, ont été évaluées selon une méthode statique par bullage d'azote. et comparées à des compositions solubilisantes de l'état de la technique, à savoir :

- La composition de n-hexylpolyglucosides commercialisée sous le nom de marque AG 6206 par la société AKZO NOBEL (composition $X_1$),
- La composition de 2-ethylhexylpolyglucosides commercialisée sous le nom de marque AG 6202 par la société NOBEL AKZO (composition $X_2$),
- La composition de n-octylpolyglucosides/n-décylpolyglucosides commercialisée sous le nom de marque SIMUL-SOL™SL8 (composition $X_3$) par la société SEPPIC,
- Le cumène sulfonate de sodium (composition $X_4$) commercialisé sous le nom de marque ELTESOL™SC Pellets par la société IMCD France.

**1.2.1) Principe de la méthode statique par bullage d'azote pour l'évaluation du pouvoir moussant**

La mousse est formée par l'introduction d'un volume déterminé d'azote dans une solution de tensioactif à concentration fixée et en présence d'une quantité fixe de soude, à une température spécifique. Le volume de mousse généré par l'introduction du volume d'azote est mesuré à la fin de l'introduction dudit volume d'azote, puis à une durée de 30 secondes, puis de 120 secondes à l'issue de la fin de l'introduction du volume d'azote.

1.2.2) Protocole expérimental

50 cm3 d'une solution à 5 g/L en extrait sec des compositions testées sont introduits dans une éprouvette graduée thermostatée de 250 cm3 ainsi qu'une quantité de 12,5 grammes de soude. Les mesures ont été effectuées à 20°C et 60°C. Un doigt pour distribution à gaz de porosité 3 (*réf* Corning Pyrex 853-1) est positionné de telle façon que l'extrémité de l'embout fritté se trouve à un centimètre du fond de l'éprouvette. Le débit d'azote est alors précisément réglé à 50 l/h et le barbotage est réalisé pendant 15 secondes. Après cette durée, l'arrivée de l'azote est coupée et l'expérimentateur note le volume de mousse initial ainsi que le volume de mousse après 30 secondes et 120 secondes. Au moins deux essais conduisant à des résultats équivalents ont été réalisés dans des éprouvettes différentes pour une même solution de tensioactif.

**1.2.3) Expression des résulats**

Les résultats du volume de mousse observé dans l'éprouvette graduée initialement, puis à 30 secondes et à 120 secondes sont exprimés ou $cm^3$.

**1.2.4) Caractérisation du pouvoir moussant de la composition ($X_0$) selon l'invention par rapport à celui des compositions ($X_1$), ($X_2$), ($X_3$) et ($X_4$) comprenant les composés de l'état de la technique.**

**1.2. 4.1) Résultats obtenus**

Le protocole expérimental décrit dans la section 1.2.2 de la présente demande a été mis en oeuvre pour la composition de n-heptyl polyglucosides (composition $X_0$) obtenue selon le procédé décrit dans la section 1.1 de la présente demande, et pour les compositions ($X_1$), ($X_2$), ($X_3$) et ($X_4$) précédemment décrites.

Les mesures expérimentales ont été menées à deux températures différentes : à 20°C et à 60°C pour chacune des compositions décrites ci-dessus.

Les mesures expérimentales, pour chaque composition et à chaque température, ont été relevée à la fin de l'introduction du volume d'azote (t = 0), 30 secondes après la fin de l'introduction du volume d'azote (t = 30s) et 120 secondes après la fin de l'introduction du volume d'azote (t = 120s), et ont été consignées dans les tableaux 2 et 3 ci-dessous pour les mesures effectuées respectivement à 20°C et à 60°C.

**Tableau 2 :** Pouvoir moussant à 20°C

| | | Composition | | | | |
|---|---|---|---|---|---|---|
| | | ($X_0$) | ($X_1$) | ($X_2$) | ($X_3$) | ($X_4$) |
| Volume de mousse (en $cm^3$) | A t = 0 | 100 | 65 | 120 | 125 | 65 |
| | A t = 30 s | 5 | 5 | 90 | 110 | 5 |
| | A t = 120 s | 0 | 0 | 50 | 100 | 5 |

**Tableau 3 :** Pouvoir moussant à 60°C

| | | Composition | | | | |
|---|---|---|---|---|---|---|
| | | ($X_0$) | ($X_1$) | ($X_2$) | ($X_3$) | ($X_4$) |
| Volume de mousse (en $cm^3$) | A t = 0 | 10 | 30 | 70 | 140 | 45 |
| | A t = 30 s | 5 | 1 | 30 | 120 | 1 |
| | A t = 120 s | 0 | 0 | 10 | 110 | 0 |

**1.2. 4.2. Analyse des résultats.**

La composition ($X_0$) objet de la présente invention se caractérise par la génération d'une mousse très instable à 20°C puisque le volume de mousse diminue en 30 secondes de 95% de sa valeur initiale, contre 92,3% pour la composition ($X_1$), 25% pour la composition ($X_2$) et 12% pour la composition ($X_3$).

A 60°C, la composition ($X_0$) de n-heptylpolyglucosides objet de la présente invention se caractérise également par la génération d'une mousse très instable puisque le volume de mousse diminue en 30 secondes de 100 % de sa valeur initiale, contre 57,1% pour la composition ($X_2$) et 14% pour la composition ($X_3$). A 60°C, la composition ($X_0$) par la génération d'un volume de mousse inférieur à celui généré par les compositions de l'état de la technique.

**1.3) Evaluation des propriétés mouillantes de n-heptylpolyglucosides**

Les propriétés mouillantes de la composition ($X_0$) de n-heptylpolyglucosides, obtenue selon le procédé précédemment décrit, ont été évaluées selon une méthode d'évaluation sur un disque de coton, adaptée des normes ISO 8022, édition de 1990, et N FT 73420.

**1.3.1) Principe de la méthode sur disque de coton pour l'évaluation du pouvoir mouillant**

Cette méthode a pour objet de déterminer la mouillabilité d'un tensioactif par rapport à un support textile, en l'occurrence du coton écru. Le pouvoir mouillant est apprécié par la mesure de la durée de mouillage d'un disque de coton écru placé au sein d'une solution de tensioactifs à une concentration définie, en présence d'une quantité de soude définie.

**1.3.2) Protocole expérimental**

700 cm$^3$ d'une solution à 5 g/L en extrait sec des compositions testées dans de l'eau distillée, sont placés dans un bêcher thermostaté à la température souhaitée en présence de 35 grammes de soude. Un disque de coton écru répondant à la norme NFT 73-406 (30 mm de diamètre) et fourni par la société MORTELECQUE, est introduit dans la solution précédemment préparée à l'aide d'une pince d'immersion spécifique à ce test. La durée de mouillage est déterminée expérimentalement à l'aide d'un chronomètre déclenché au moment où la partie inférieure du disque touche la solution et arrêté au moment où le disque s'enfonce par lui-même dans la solution, de façon à obtenir une durée de mouillage. Dix mesures consécutives de la durée de mouillage ont été réalisées avec la même solution pour chaque composition en prenant cependant soin de jeter les disques de coton utilisé après chaque mesure.

**1.3.3- Expression des résulats**

Le pouvoir mouillant est exprimé par une durée $t_m$ en seconde correspondant à la moyenne des dix mesures effectuées pour chacune des compositions testées.

**1.3.4) Caractérisation du pouvoir mouillant de la composition ($X_0$) selon l'invention par rapport à celui des compositions ($X_1$), ($X_2$), ($X_3$) et ($X_4$) comprenant les composés de l'état de la technique.**

**1.3. 4.1.) Résultats obtenus**

Le protocole expérimental décrit dans la section 1.3.2 de la présente demande a été mis en oeuvre pour la composition de n-heptyl polyglucosides (composition $X_0$) obtenue selon le procédé décrit dans la section 1.1 de la présente demande, et pour les compositions ($X_1$), ($X_2$), ($X_3$) et ($X_4$) décrites précédemment.
Les mesures expérimentales ont été menées à deux températures différentes : à 20°C et à 60°C pour chacune des compositions décrites ci-dessus.
Les mesures expérimentales des durées de mouillage $t_m$, mesurées pour chaque composition et à 20°C et à 60°C, ont été relevées et consignées dans le tableau 4.

<u>Tableau 4 :</u> pouvoir mouillant à 20°C et à 60°C

| | | Composition | | | | |
|---|---|---|---|---|---|---|
| | | ($X_0$) | ($X_1$) | ($X_2$) | ($X_3$) | ($X_4$) |
| Pouvoir mouillant sur disque de coton (exprimée en durée de mouillage) | à 20°C | > 300s | > 300s | 25s | 11s | > 300s |
| | à 60°C | 155s | > 300s | 49 | 23s | > 300s |

**1.3.4.2) Analyse des résultats.**

A 20°C, la composition ($X_0$) de n-heptylpolyglucosides objet de la présente invention se caractérise par un pouvoir mouillant faible, identique à celui de la composition ($X_1$) et à celui de la composition ($X_4$), mais inférieur à ceux des composition ($X_2$) et.
A 60°C, le pouvoir mouillant de la composition ($X_0$) est meilleur, alors que celui des compositions ($X_1$) et ($X_4$) reste faible et que ceux des compositions ($X_2$) et ($X_3$) sont en diminution.

**1.4) Evaluation des propriétés solubilisantes de n-heptylpolyglucosides en milieu sodique**

Les propriétés solubilisantes en milieu sodique de la composition ($X_0$) de n-heptylpolyglucosides, obtenue selon le procédé précédemment décrit, ont été évaluées comparativement aux compositions ($X_1$), ($X_2$), ($X_3$) et ($X_4$) de l'état de la technique telles que décrites précédemment selon les méthodes d'évaluation décrites ci-dessous pour différents tensioactifs non-ioniques et à différentes concentration de soude.

**1.4.1- Evaluation du pouvoir solubilisant en milieu sodique pour plusieurs ratios massiques de tensioactifs à solubiliser/composition solubilisante.**

**1.4.1.1- Principe de la méthode**

Cette méthode a pour objet de déterminer le pouvoir solubilisant d'une composition tensioactive en milieu sodique pour un tensioactif non-ionique insoluble en milieu sodique fixé par rapport à des compositions tensioactives de l'état de la technique.

**1.4.1.2- Protocole expérimental**

On introduit dans un flacon de verre de 120 cm3 une quantité d'un gramme d'un tensioactif non-ionique $(Ti)$ à solubiliser, une quantité de $X_1$ gramme de la composition tensioactive solubilisante $(Xi)$ à tester, une quantité de $y_1$ grammes de soude et une quantité d'eau distillée complémentaire de façon à obtenir une solution de 100 cm3. Un barreau magnétique aimanté est introduit dans le flacon de verre qui est ensuite placé sous agitation magnétique à une vitesse de 100 tours/minute pendant une durée de 1 heure à une température de 20°C.

**1.4.1.3- Expression des résultats**

L'aspect visuel de la solution obtenue selon le protocole de la section 1.4.1.2 de la présente demande est noté par l'expérimentateur et qualifié de « limpide » ou de « trouble » selon les cas.

**1.4.1.4- caractérisation du pouvoir solubilisant en milieu sodique de la composition $(X_0)$ selon l'invention par rapport à celui des compositions $(X_1)$, $(X_2)$, $(X_3)$ et $(X_4)$ comprenant les composés de l'état de la technique.**

**Résultats obtenus**

Le protocole expérimental décrit dans la section 1.4.1.2 de la présente demande **a** été mis en oeuvre pour la composition $(X_0)$ selon l'invention et pour les compositions tensioactives solubilisantes $(X_1)$, $(X_2)$, $(X_3)$ et $(X_4)$ de l'état de la technique.

Le protocole expérimental décrit dans la section 1.4.2. de la présente demande a été mis en oeuvre pour les tensioactifs non-ioniques $(Ti)$ suivant :

- Composition d'alcools polyéthoxylés $(T_1)$, commercialisée sous le nom de marque SIMULSOL™OX1004L par la société SEPPIC, résultant de la réaction d'un équivalent molaire d'un alcool commercialisé sous le nom de marque EXXAL™10 (numéro CAS : 68526-85-2) par la société EXXONMOBIL, comprenant un mélange de n-nonanol, de n-décanol, de n-undécanol, d'isononanol, d'isodécanol, d'isoundécanol, avec 4 équivalents molaires d'oxyde d'éthylène,

- Composition d'alcools polyéthoxylés $(T_2)$, commercialisée sous le nom de marque SIMULSOLT™OX1006L par la société SEPPIC, résultant de la réaction d'un équivalent molaire d'un alcool commercialisé sous le nom de marque EXXAL™10 (numéro CAS : 68526-85-2) par la société EXXONMOBIL, comprenant un mélange de n-nonanol, de n-décanol, de n-undécanol, d'isononanol, d'isodécanol, d'isoundécanol, avec 6 équivalents molaires d'oxyde d'éthylène,

- Composition d'alcools polyéthoxylés $(T_3)$, commercialisé sous le nom de marque SIMULSOL™OX1309L par la société SEPPIC, résultant de la réaction d'un équivalent molaire d'un alcool commercialisé sous le nom de marque EXXAL™13 (numéro CAS : 68256-86-3) par la société EXXONMOBIL, comprenant un mélange de n-undécanol, de n-dodécanol, de n-tridécanol, de n-tétradécanol, d'isoundécanol, d'isododécanol d'isotridécanol, avec 9 équivalents molaires d'oxyde d'éthylène,

- Composition d'alcools polyéthoxylés $(T_4)$, préparée par la réaction entre 1 équivalent molaire de n-décanol avec 4 équivalents molaires d'oxyde d'éthylène en présence de potasse comme catalyseur basique.

Les mesures expérimentales ont été menées en présence de quantités $y_1$ différentes de soude de façon à obtenir des teneurs massiques de 10%, 20%, 30% et 48% pour chacune des quantités $X_1$ des différentes compositions solubilisantes $(X_0)$, $(X_1)$, $(X_2)$, $(X_3)$ et $(X_4)$ testées, et pour chacun des tensioactifs non-ioniques $(T_1)$, $(T_2)$, $(T_3)$ et $(T_4)$ décrits ci-dessus. La quantité $X_1$ des différentes compositions solubilisantes $(X_0)$, $(X_1)$, $(X_2)$, $(X_3)$ et $(X_4)$ testées est déterminée de façon à atteindre des ratios massiques Composition $(Ti)$ / compositions $(Xi)$ $(Ti / Xi)$ égaux à 1/1, à 1/2 et à 1/5.

Les aspects des solutions préparées par la mise en oeuvre du protocole opératoire pour chacune des compositions $(X_0)$, $(X_1)$, $(X_2)$, $(X_3)$ et $(X_4)$ utilisées pour solubiliser les compositions $(T_1)$, $(T_2)$, $(T_3)$ et $(T_4)$ ont été relevés par l'expérimentateur et consignés respectivement dans les tableaux 5, 6, 7 et 8 ci-dessous.

**Tableau 5 :** Aspect de solutions comprenant la composition tensioactive non-ionique (T1) en présence des compositions $(X_0)$, $(X_1)$, $(X_2)$, $(X_3)$ et $(X_4)$ à 20°C

| | | | | Aspect des compositions | | | | |
|---|---|---|---|---|---|---|---|---|
| | | | | $X_0$ | $X_1$ | $X_2$ | $X_3$ | $X_4$ |
| Quantité massique de soude (%massique) | 10% | Ratio massique($T_1$ / Xi) | 1/1 | L | T | T | L | T |
| | | | 1/2 | L | L | L | L | L |
| | | | 1/5 | L | L | L | L | L |
| | 20% | Ratio massique($T_1$ / Xi) | 1/1 | L | T | T | T | T |
| | | | 1/2 | L | L | L | T | T |
| | | | 1/5 | L | L | L | L | T |
| | 48% | Ratio massique($T_1$ / Xi) | 1/1 | L | T | T | T | T |
| | | | 1/2 | L | T | T | T | T |
| | | | 1/5 | L | L | L | T | T |
| (L : Limpide ; T : Trouble). | | | | | | | | |

**Tableau 6 :** Aspect de solutions comprenant la composition tensioactive non-ionique (T2) en présence des compositions $(X_0)$, $(X_1)$, $(X_2)$, $(X_3)$ et $(X_4)$ à 20°C.

| | | | | Aspect des compositions | | | | |
|---|---|---|---|---|---|---|---|---|
| | | | | $X_0$ | $X_1$ | $X_2$ | $X_3$ | $X_4$ |
| Quantité massique de soude (%massique) | 10% | Ratio massique ($T_2$ / Xi) | 1/1 | L | T | L | L | T |
| | | | 1/2 | L | L | L | L | L |
| | | | 1/5 | L | L | L | L | L |
| | 20% | Ratio massique ($T_2$ / Xi) | 1/1 | L | T | T | T | T |
| | | | 1/2 | L | L | T | T | T |
| | | | 1/5 | L | L | L | L | T |
| | 48% | Ratio massique ($T_2$ / Xi) | 1/1 | L | T | T | T | T |
| | | | 1/2 | L | T | T | T | T |
| | | | 1/5 | L | L | L | L | T |
| (L : Limpide ; T : Trouble). | | | | | | | | |

**Tableau 7 :** Aspect de solutions comprenant la composition tensioactive non-ionique (T3) en présence des compositions ($X_0$), ($X_1$), ($X_2$), ($X_3$) et ($X_4$) à 20°C.

| | | | | Aspect des compositions | | | | |
|---|---|---|---|---|---|---|---|---|
| | | | | $X_0$ | $X_1$ | $X_2$ | $X_3$ | $X_4$ |
| Quantité massique de soude (%massique) | 10% | Ratio massique ($T_3 / Xi$) | 1/1 | L | T | L | L | T |
| | | | 1/2 | L | L | L | L | L |
| | | | 1/5 | L | L | L | L | L |
| | 48% | Ratio massique ($T_3 / Xi$) | 1/1 | L | T | L | L | T |
| | | | 1/2 | L | T | L | L | T |
| | | | 1/5 | L | L | L | L | T |
| (L : Limpide ; T : Trouble). | | | | | | | | |

**Tableau 8 :** Aspect de solutions comprenant la composition tensioactive non-ionique (T4) en présence des compositions ($X_0$), ($X_1$), ($X_2$), ($X_3$) et ($X_4$) à 20°C.

| | | | | Aspect des compositions | | | | |
|---|---|---|---|---|---|---|---|---|
| | | | | $X_0$ | $X_1$ | $X_2$ | $X_3$ | $X_4$ |
| | 10% | Ratio massique ($T_4 / Xi$) | 1/1 | L | T | T | T | T |
| | | | 1/2 | L | L | L | L | L |
| | | | 1/5 | L | L | L | L | L |
| Quantité massique de soude (%massique) | 20% | Ratio massique ($T_4 / Xi$) | 1/1 | L | T | T | T | T |
| | | | 1/2 | L | L | L | T | T |
| | | | 1/5 | L | L | L | L | T |
| | 48% | Ratio massique ($T_4 / Xi$) | 1/1 | L | T | T | T | T |
| | | | 1/2 | L | T | T | T | T |
| | | | 1/5 | L | L | L | T | T |
| (L : Limpide ; T : Trouble). | | | | | | | | |

**Analyse des résultats.**

La comparaison entre les performances solubilisantes observées pour les compositions se caractérisant par un faible pouvoir moussant, à savoir la composition ($X_0$), la composition ($X_1$) et la composition ($X_4$) montre que quelle que soit la quantité de soude présente dans la solution détergente alcaline préparée, la composition ($X_0$) comprenant des n-heptylpolyglucosides objets de la présente invention se caractérise par un pouvoir solubilisant plus important que celui observé par la composition ($X_1$). La comparaison entre les performances solubilisantes observées pour la composition ($X_0$) et pour la composition ($X_2$) montre que la composition ($X_0$) se caractérise par un pouvoir solubilisant plus important celui de la composition ($X_2$).

**1.4.2- Evaluation du pouvoir solubilisant en milieu sodique par détermination de la quantité minimale de composition solubilisante pour solubiliser une quantité fixe de tensioactifs à solubiliser.**

**1.4.2.1- Principe de la méthode**

Cette méthode a pour objet de déterminer le pouvoir solubilisant d'une composition tensioactive en milieu sodique pour un tensioactif non-ionique insoluble en milieu sodique fixé par rapport à des compositions tensioactives de l'état de la technique.

Dans cette méthode, la quantité de tensioactif non-ionique insoluble en milieu sodique est fixée à hauteur de 5% pour 100% de la masse de chaque milieu sodique aqueux retenu, et l'expérimentateur détermine par ajout progressif de la composition tensioactive solubilisante la quantité minimale de celle-ci pour obtenir une solution sodique limpide. La méthode est mise en oeuvre pour les compositions selon l'invention et pour des compositions tensioactives de l'état de la technique.

**1.4.2.2- Protocole expérimental**

On introduit dans un flacon de verre de 120 cm3 une quantité de 5 grammes du tensioactif non-ionique (Ti) à solubiliser et une quantité de 95 grammes d'un mélange constitué d'eau distillée et de $y_1$ grammes de soude.

Un barreau magnétique aimanté est introduit dans le flacon de verre qui est ensuite placé sous agitation magnétique à une vitesse de 100 tours/minute pendant une durée de 1 heure à une température de 20°C.

On introduit par la suite de façon progressive la composition tensioactive solubilisante (Xi) à tester et l'expérimentateur détermine la quantité minimale de $X_1$ gramme de ladite composition tensioactive solubilisante (Xi) nécessaire pour obtenir une solution aqueuse sodique limpide.

**1.4.2.3- Expression des résultats**

Lorsque l'aspect visuel de la solution obtenue selon le protocole de la section 1.4.2.2 de la présente demande est limpide, l'expérimentateur note la quantité $X_1$ de la composition tensioactive solubilisante (Xi) ajoutée pour atteindre cet aspect limpide.

**1.4.2.4- caractérisation du pouvoir solubilisant en milieu sodique de la composition ($X_0$)selon l'invention par rapport à celui des compositions ($X_1$), ($X_2$) et ($X_3$) comprenant les composés de l'état de la technique.**

**Résultats obtenus**

Le protocole expérimental décrit dans la section 1.4.2.2 de la présente demande a été mis en oeuvre pour les compositions tensioactives solubilisantes ($X_0$), ($X_1$), ($X_2$) et ($X_3$) et pour les compositions tensioactives non-ioniques ($T_5$) et ($T_6$) telles que décrites ci-dessous :

- Composition d'alcools polyéthoxylés ($T_5$), préparée par la réaction entre 1 équivalent molaire d'un mélange comprenant, pour 100% de sa masse, 50% massique de n-octanol et 50% massique de n-décanol, avec 4 équivalents molaires d'oxyde d'éthylène en présence de potasse comme catalyseur basique,
- Composition d'alcools polyéthoxylés ($T_6$), préparée par la réaction entre 1 équivalent molaire d'un mélange comprenant, pour 100% de sa masse, 85% massique de n-décanol et 15% massique de n-dodécanol, avec 4 équivalents molaires d'oxyde d'éthylène en présence de potasse comme catalyseur basique.

Les mesures expérimentales ont été menées selon le protocole expérimental décrit dans la section 1.4.2.2 en présence de quantités $y_1$ différentes de soude de façon à obtenir des teneurs massiques en soude de 10% et de 40% pour chacune des compositions ($T_5$) et ($T_6$).

Les quantités minimales des compositions ($X_0$), ($X_1$), ($X_2$) et ($X_3$) nécessaires pour obtenir une solution limpide ont été relevées par l'expérimentateur et consignées dans le tableau 9 ci-dessous.

**Tableau 9 :** Quantités minimales de compositions $X_0$), ($X_1$), ($X_2$) et ($X_3$) nécessaires pour solubiliser des solutions sodiques comprenant les compositions tensioactives non ioniques ($T_5$) et ($T_6$) à 20°C.

| Composition (Ti) à solubiliser | Quantité de soude | Quantité minimale de composition (Xi) en gramme pour obtenir une solution limpide selon le protocole décrit dans la section 1.4.2.2 | | | |
|---|---|---|---|---|---|
| | | $X_0$ | $X_1$ | $X_2$ | $X_3$ |
| (T5) | 10 % | 3,3 g | 3,8 g | 4,1 g | 4,9 g |
| | 40 % | 7,6 g | 13,0 g | 9,3 g | 12,4 g |
| (T6) | 10 % | 3,02 g | 3,62 g | 3,53 g | 4,16 g |
| | 40 % | 6,70 g | 12,79 g | 9,20 g | 10,92 g |

**Analyse des résultats.**

La comparaison entre les performances solubilisantes observées pour les compositions se caractérisant par un faible pouvoir moussant, à savoir la composition $(X_0)$ et la composition $(X_1)$ montre que quelle que soit la quantité de soude présente dans la solution détergente alcaline préparée, la composition $(X_0)$ comprenant des n-heptylpolyglucosides objets de la présente invention se caractérise par un pouvoir solubilisant plus important que celui observé par la composition $(X_1)$. La comparaison entre les performances solubilisantes observées pour la composition $(X_0)$ et pour la composition $(X_2)$ montre que la composition $(X_0)$ se caractérise par un pouvoir solubilisant plus important celui de la composition $(X_2)$.

**1.5) Evaluation des propriétés solubilisantes de n-heptylpolyglucosides en milieu électrolytique**

Les propriétés solubilisantes en milieu électrolytique de la composition $(X_0)$ de n-heptylpolyglucosides, obtenue selon le procédé précédemment décrit, ont été évaluées comparativement aux compositions $(X_1)$, $(X_2)$, $(X_3)$ et $(X_4)$ de l'état de la technique telle que décrites précédemment selon les méthodes d'évaluation décrites ci-dessous pour différents tensioactifs non-ioniques et à différentes concentration de métasilicate de sodium.

**1.5.1) - Evaluation du pouvoir solubilisant en milieu électrolytique pour plusieurs ratios massiques de tensioactifs à solubiliser /composition solubilisante**

**1.5.1.1- Principe de la méthode**

Cette méthode a pour objet de déterminer le pouvoir solubilisant d'une composition tensioactive en milieu électrolytique pour un tensioactif non-ionique insoluble en milieu électrolytique fixé par rapport à des compositions tensioactives de l'état de la technique.

**1.5.1.2 - Protocole expérimental**

On introduit dans un flacon de verre de 120 cm$^3$ une quantité d'un gramme du tensioactif non-ionique (Ti) à solubiliser, une quantité de $X_2$ gramme de la composition tensioactive solubilisante (Xi) à tester, une quantité de $y_2$ grammes de métasilicate de sodium et une quantité d'eau distillée complémentaire de façon à obtenir une solution de 100 cm3. Un barreau magnétique aimanté est introduit dans le flacon de verre qui est ensuite placé sous agitation magnétique à une vitesse de 100 tours/minute pendant une durée de 1 heure à une température de 20°C.

**1.5.2.3- Expression des résultats**

L'aspect visuel de la solution obtenue selon le protocole de la section 1.5.1.2 de la présente demande est noté par l'expérimentateur et qualifié de « limpide » ou de « trouble » selon les cas.

**1.5.1.4- caractérisation du pouvoir solubilisant en milieu électrolytique de la composition $(X_0)$ selon l'invention par rapport à celui des compositions $(X_1)$, $(X_2)$, $(X_3)$ et $(X_4)$ comprenant les composés de l'état de la technique.**

**Résultats obtenus**

Le protocole expérimental décrit dans la section 1.5.1.2 de la présente demande a été mis en oeuvre pour les compositions tensioactives solubilisantes $(X_0)$, $(X_1)$, $(X_2)$, $(X_3)$ et $(X_4)$ et pour les compositions tensioactives non-ioniques $(T_1)$, $(T_2)$, $(T_3)$ et $(T_4)$, telles que décrites précédemment.

Les mesures expérimentales ont été menées en présence de quantités $y_2$ différentes de métasilicate de sodium de façon à obtenir des teneurs massiques de 10%, 20% et 30% pour chacune des quantités $x_2$ des différentes compositions solubilisantes $(X_1)$, $(X_2)$, $(X_3)$ et $(X_4)$ testées, et pour chacun des tensioactifs non-ioniques $(T_1)$, $(T_2)$, $(T_3)$ et $(T_4)$ décrits ci-dessus. La quantité $X_2$ des différentes compositions solubilisantes $(X_1)$, $(X_2)$, $(X_3)$ et $(X_4)$ testées est déterminée de façon à atteindre des ratios massiques composition (Ti)/compositions (Xi) égal à 1/1, à 1/2 et à 1/5.

**Tableau 10 :** Aspect de solutions comprenant la composition tensioactive non-ionique ($T_1$) en présence des compositions ($X_0$), ($X_1$), ($X_2$), ($X_3$) et ($X_4$) à 20°C.

| Quantité massique de métasilicate de sodium | Ratio massique Composition ($T_1$)/compositions (Xi) | Aspect observé | | | | |
|---|---|---|---|---|---|---|
| | | ($X_0$) | ($X_1$) | ($X_2$) | ($X_3$) | ($X_4$) |
| 10% | 1/1 | L | T | T | T | T |
| | 1/2 | L | L | L | L | L |
| | 1/5 | L | L | L | L | L |
| 20% | 1/1 | L | T | T | T | T |
| | 1/2 | L | L | T | T | L |
| | 1/5 | L | L | L | L | L |
| 30% | 1/1 | L | T | T | T | T |
| | 1/2 | L | T | T | T | T |
| | 1/5 | L | T | T | T | T |
| (L : Limpide ; T : Trouble). | | | | | | |

**Tableau 11 :** Aspect de solutions comprenant la composition tensioactive non-ionique ($T_2$) en présence des compositions ($X_0$), ($X_1$), ($X_2$), ($X_3$) et ($X_4$) à 20°C.

| Quantité massique de métasilicate de sodium | Ratio massique Composition (T2)/compositions (Xi) | Aspect observé | | | | |
|---|---|---|---|---|---|---|
| | | ($X_0$) | ($X_1$) | ($X_2$) | ($X_3$) | ($X_4$) |
| 10% | 1/1 | L | T | L | L | L |
| | 1/2 | L | L | L | L | L |
| | 1/5 | L | L | L | L | L |
| 20% | 1/1 | L | T | T | T | T |
| | 1/2 | L | L | L | L | L |
| | 1/5 | L | L | L | L | L |
| 30% | 1/1 | L | T | T | T | T |
| | 1/2 | L | T | T | T | T |
| | 1/5 | L | T | T | T | T |
| (L : Limpide ; T : Trouble). | | | | | | |

**Tableau 12 :** Aspect de solutions comprenant la composition tensioactive non-ionique ($T_3$) en présence des compositions ($X_0$), ($X_1$), ($X_2$), ($X_3$) et ($X_4$) à 20°C.

| Quantité massique de métasilicate de sodium | Ratio massique Composition (T3)/compositions (Xi) | Aspect observé | | | | |
|---|---|---|---|---|---|---|
| | | ($X_0$) | ($X_1$) | ($X_2$) | ($X_3$) | ($X_4$) |
| 10% | 1/1 | L | T | L | L | L |
| | 1/2 | L | L | L | L | L |
| | 1/5 | L | L | L | L | L |

(suite)

| Quantité massique de métasilicate de sodium | Ratio massique Composition (T3)/compositions (Xi) | Aspect observé | | | | |
|---|---|---|---|---|---|---|
| | | $(X_0)$ | $(X_1)$ | $(X_2)$ | $(X_3)$ | $(X_4)$ |
| 30% | 1/1 | L | T | T | T | T |
| | 1/2 | L | T | T | T | T |
| | 1/5 | L | T | T | T | T |
| (L : Limpide ; T : Trouble). | | | | | | |

**Tableau 13 :** Aspect de solutions comprenant la composition tensioactive non-ionique (T4) en présence des compositions $(X_0)$, $(X_1)$, $(X_2)$, $(X_3)$ et $(X_4)$ à 20°C.

| Quantité massique de métasilicate de sodium | Ratio massique Composition $(T_4)$/compositions (Xi) | Aspect observé | | | | |
|---|---|---|---|---|---|---|
| | | $(X_0)$ | $(X_1)$ | $(X_2)$ | $(X_3)$ | $(X_4)$ |
| 10% | 1/1 | L | T | L | L | L |
| | 1/2 | L | T | L | L | T |
| | 1/5 | L | T | L | L | T |
| 20% | 1/1 | L | T | T | T | L |
| | 1/2 | L | T | T | T | T |
| | 1/5 | L | T | L | T | T |
| 30% | 1/1 | L | T | T | T | T |
| | 1/2 | L | T | T | T | T |
| | 1/5 | L | T | T | T | T |
| (L : Limpide ; T : Trouble). | | | | | | |

**Analyse des résultats obtenus**

La comparaison entre les performances solubilisantes observées pour les compositions se caractérisant par un faible pouvoir moussant, à savoir la composition (X0), la composition (X1) et la composition (X4), montre que pour une quantité de 10 % massique de silicate de sodium, un seul équivalent massique de composition (X0) est nécessaire pour obtenir une solution détergente alcaline limpide pour l'ensemble des compositions tensioactives non-ioniques (T1), (T2), (T3) et (T4). contrairement aux autres compositions.

La comparaison entre les performances solubilisantes observées pour la composition $(X_0)$ et pour la composition (X2) montre que, pour une quantité de silicate de sodium présente dans une proportion de 30% massique dans la composition détergente alcaline, l'utilisation de 5 équivalents massiques de la composition (X2) ne parvient pas à solubiliser les compositions tensioactives non-ionique (T1), (T2), (T3) et (T4), alors qu'un seul équivalent massique de la composition $(X_0)$ est nécéssaire pour solubiliser les compositions tensioactives non-ioniques (T1), (T2), (T3) et (T4).

**1.5.2- Evaluation du pouvoir solubilisant en milieu électrolytique par détermination de la quantité minimale de composition solubilisante pour solubiliser une quantité fixe de tensioactifs à solubiliser.**

**1.5.2.1- Principe de la méthode**

Cette méthode a pour objet de déterminer le pouvoir solubilisant d'une composition tensioactive en milieu électrolytique pour un tensioactif non-ionique insoluble en milieu électrolytique fixé par rapport à des compositions tensioactives de l'état de la technique.

Dans cette méthode, la quantité de tensioactif non-ionique insoluble en milieu électrolytique est fixée à hauteur de 5% pour 100% de la masse de chaque milieu électrolytique aqueux retenu, et l'expérimentateur détermine par ajout progressif de la composition tensioactive solubilisante la quantité minimale de celle-ci pour obtenir une solution électrolytique limpide. La méthode est mise en oeuvre pour les compositions selon l'invention et pour des compositions tensioactives de l'état de la technique.

### 1.5.2.2- Protocole expérimental

On introduit dans un flacon de verre de 120 cm3 une quantité de 5 grammes du tensioactif non-ionique (Ti) à solubiliser et une quantité de 95 grammes d'un mélange constitué d'eau distillée et d'une quantité de $y_2$ grammes de métasilicate de sodium.

Un barreau magnétique aimanté est introduit dans le flacon de verre qui est ensuite placé sous agitation magnétique à une vitesse de 100 tours/minute pendant une durée de 1 heure à une température de 20°C.

On introduit par la suite de façon progressive la composition tensioactive solubilisante (Xi) à tester et l'expérimentateur détermine la quantité minimale de $X_1$ gramme de ladite composition tensioactive solubilisante (Xi) nécessaire pour obtenir une solution aqueuse électrolytique limpide.

### 1.5.2.3- Expression des résultats

Lorsque l'aspect visuel de la solution obtenue selon le protocole de la section 1.5.2.2 de la présente demande est limpide, l'expérimentateur note la quantité $X_1$ de la composition tensioactive solubilisante (Xi) ajoutée pour atteindre cet aspect limpide.

### 1.5.2.4- caractérisation du pouvoir solubilisant en milieu électrolytique de la composition ($X_0$) selon l'invention par rapport à celui des compositions ($X_1$), ($X_2$) et ($X_3$) comprenant les composés de l'état de la technique.

### Résultats obtenus

Le protocole expérimental décrit dans la section 1.5.2.2 de la présente demande a été mis en oeuvre pour les compositions tensioactives solubilisantes ($X_0$), ($X_1$), ($X_2$) et ($X_3$) et pour les compositions tensioactives non-ioniques ($T_5$) et ($T_6$) telles que décrites précédemment.

Les mesures expérimentales ont été menées selon le protocole expérimental décrit dans la section 1.5.2.2 en présence de quantités $y_2$ différentes de métasilicate de sodium de façon à obtenir des teneurs massiques en métasilicate de sodium de 10% et de 20% pour chacune des compositions ($T_5$) et ($T_6$).

Les quantités minimales des compositions ($X_0$), ($X_1$), ($X_2$) et ($X_3$) nécessaires pour obtenir une solution limpide ont été relevées par l'expérimentateur et consignées dans le tableau 14 ci-dessous.

<u>Tableau 14 :</u> Quantités minimales de compositions ($X_0$), ($X_1$), ($X_2$) et ($X_3$) nécessaires pour solubiliser des solutions électrolytiques comprenant les compositions tensioactives non ioniques ($T_5$) et ($T_6$) à 20°C.

| Composition ($T_i$) à solubiliser | Quantité de métasilicate de sodium | Quantité minimale de composition ($X_i$) en gramme pour obtenir une solution limpide selon le protocole décrit dans la section 1.5.2.2 | | | |
|---|---|---|---|---|---|
| | | $X_0$ | $X_1$ | $X_2$ | $X_3$ |
| ($T_5$) | 10% | 2,39 g | 3,70 g | 2,76 g | 2,92 g |
| | 20% | 4,13 g | 5,04 g | 6,80 g | 6,08 g |
| ($T_6$) | 10% | 2,77 g | 4,19 g | 3,20 g | 3,60 g |
| | 20% | 3,84 g | 4,78 g | 6,17 g | 6,84 g |

### Analyse des résultats obtenus

La comparaison entre les performances solubilisantes observées pour les compositions se caractérisant par un faible pouvoir moussant, à savoir la composition ($X_0$) et la composition ($X_1$), montre que quelle que soit la quantité de métasilicate de sodium présente dans la solution détergente préparée, la composition ($X_0$) comprenant des n-heptylpolyglucosides objets de la présente invention se caractérise par un pouvoir solubilisant plus important que celui observé pour la composition ($X_1$), puisque la quantité minimale nécessaire pour obtenir une solution limpide est moindre pour la composition ($X_0$) que pour la composition ($X_1$).

La comparaison entre les performances solubilisantes observées pour la composition ($X_0$) et pour les compositions ($X_2$) et ($X_3$) montre que quelle que soit la quantité de métasilicate de sodium présente dans la solution détergente préparée, la composition ($X_0$) comprenant des n-heptylpolyglucosides objets de la présente invention se caractérise par un pouvoir solubilisant plus important que celui observé pour les compositions ($X_2$) et ($X_3$), puisque la quantité minimale nécessaire pour obtenir une solution limpide est moindre pour la composition ($X_0$) que pour les compositions ($X_2$) et ($X_3$).

**1.6) Conclusions**

La composition ($X_0$) comprenant des n-heptylpolyglucosides objets de la présente invention montre des propriétés peu moussantes et solubilisatrice améliorées, en milieu alcalin et électrolytique, même en fortes proportions, par rapport aux agents solubilisants connus dans l'état de la technique.

**2) Compositions détergentes alcalines aqueuses**

**2.1. Nettoyant industriel pour sols**

2.1.1 Préparation de la composition de nettoyage industriel pour sols.

| Ingrédients | Teneur massique |
|---|---|
| SIMULSOL™ NW 900[1] | 5% |
| Composition ($X_0$) | 4% |
| DOWANOL™ DPM[2] | 2% |
| D-Limonène | 3% |
| Gluconate de sodium | 5% |
| DEQUESTTM3000S[3] | 2% |
| Solution de soude à 5% | qs pH = 13 |
| Eau | qs 100% |
| Parfum | qs |
| Colorant | qs |
| (1) SIMULSOL™ NW 900 : composition tensioactive détergente commercialisée par la société SEPPIC, comprenant des alcools polyéthoxylés résultant de la réaction d'un équivalent molaire d'un alcool commercialisé sous le nom de marque EXXAL™10 avec 9 équivalents molaires d'oxyde d'éthylène. (2) DOWANOL™ DPM : DiPropyleneGlycol Mono-Methyl Ether commercialise par la société DOW CHEMICALS (3) DEQUEST™ 3000 S : Phosphonate de sodium commercialisé par la société MONSANTO | |

Mode opératoire de préparation de la composition détergente alcaline : chaque ingrédient est introduit successivement dans une cuve de mélange sous agitation mécanique modérée, à température ambiante, jusqu'à l'obtention d'une composition homogène et limpide. L'agitation est maintenue pendant 30 minutes à 20°C puis le colorant et le parfum sont alors introduits. La composition obtenue présente un pH mesuré à 12,9, reste limpide et homogène après stockage pendant une durée d'un mois à 40°C et limpide et homogène après stockage pendant une durée d'un mois à 5°C.

**2.1.2 Procédé de nettoyage mettant en oeuvre la composition préparée en 2.1.1 :**

Une dilution à 10% dans l'eau de la composition préparée en 2.1.1 est préparée à température ambiante, puis appliquée sur un sol carrelé souillé par des salissures d'huile et de graisse, par l'intermédiaire d'une machine de nettoyage de sol. Le sol ainsi imprégné par la composition préparée en 2.1.1 est ensuite rincé avec de l'eau chaude (60°C) sous pression par l'intermédiaire d'une lance d'arrosage.

**2.2. Composition nettoyante pour voitures et camions**

**2.2.1 Préparation de la composition de nettoyage pour voitures et camions.**

| Ingrédients | Teneur massique |
|---|---|
| SIMULSOL™OX1006L[4] | 5% |
| Composition ($x_0$) | 5% |
| DOWANOL™ DPM[2] | 5% |
| Gluconate de sodium | 5% |
| Métasilicate de sodium anhydre | 0,3% |
| Potasse solide | qs pH = 12 |
| Nitrolotriacétate de sodium | 5% |
| Eau | qs 100% |
| Parfum | qs |
| Colorant | qs |
| (4) SIMULSOL™ OX1006L : composition tensioactive détergente commercialisée par la société SEPPIC, comprenant des alcools polyéthoxylés résultant de la réaction d'un équivalent molaire d'un alcool commercialisé sous le nom de marque EXXAL™ 10 avec 6 équivalents molaires d'oxyde d'éthylène. | |

<u>Mode opératoire de préparation de la composition de nettoyage pour voitures et camions :</u> chaque ingrédient est introduit successivement dans une cuve de mélange sous agitation mécanique vigoureuse, à température ambiante, jusqu'à l'obtention d'une composition homogène et limpide. L'agitation est maintenue pendant 30 minutes à 20°C puis le colorant et le parfum sont alors introduits. La composition obtenue présente un pH mesuré à 12,1, reste limpide et homogène après stockage pendant une durée d'un mois à 20°C.

<u>2.2.2 Procédé de nettoyage mettant en oeuvre la composition préparée en 2.2.1 :</u>

Une dilution à 10% dans l'eau de la composition préparée en 2.1.1 est préparée à température ambiante, puis appliquée à une température de 60°C sur la carrosserie d'un véhicule automobile souillé par de la boue et de la graisse, par l'intermédiaire d'une lance d'arrosage à faible pression. Le rinçage du véhicule imprégné par la dilution de la composition nettoyante préparée en 2.2.1 est réalisé par de l'eau à 60°C sous haute pression (100 bars). Le véhicule ainsi nettoyé ne présente plus de salissures sur ses parois et montre un aspect brillant.

**2.2.3 Nettoyage de jantes en aluminium de voitures ou de camions.**

Le procédé de nettoyage décrit en 2.2.2 est utilisé pour nettoyer des jantes en aluminium de voitures ou de camions souillées par de l'huile et de la graisse, mais en utilisant un dilution à 15% massique dans l'eau de la composition préparée en 2.2.1.

**2.3. Composition nettoyante pour fours et grilles de cuissons**

**2.3.1 Préparation de la composition de nettoyage pour fours et grilles de cuisson.**

| Ingrédients | Teneur massique |
|---|---|
| SIMULSOL™OX1309L[5] | 2% |

(suite)

| Ingrédients | Teneur massique |
|---|---|
| Composition ($X_0$) | 2% |
| SOLAGUM™SF306[6] | 6% |
| Soude à 100% | 25% |
| Eau | qs 100% |
| (5) SIMULSOL™ OX1309L : composition tensioactive détergente commercialisée par la société SEPPIC, comprenant des alcools polyéthoxylés résultant de la réaction d'un équivalent molaire d'un alcool commercialisé sous le nom de marque EXXAL™13 avec 9 équivalents molaires d'oxyde d'éthylène.<br>(6) SOLAGUM™SF 306 : composition épaississante se présentant sous la forme d'une émulsion eau-dans-huile et comprenant un polymère réticulé à base d'acrylamide et du sel de sodium du 2-acrylamido-2-méthylpropanesulfonate. | |

Mode opératoire de préparation de la composition de nettoyage pour fours et grilles de cuisson :

a) un pré-gel est préparé à 20°C par l'addition du SIMULSOL™OX1309L, puis de la composition (X0) selon l'invention dans l'eau. Le SOLAGUM™SF306 est ensuite introduit dans la solution aqueuse et mélangé jusqu'à l'obtention d'un gel de viscosité stable.
b) la soude est ensuite introduit progressivement sous agitation mécanique à une température de 20°C jusqu'à l'obtention d'un gel homogène.

Le gel obtenu à l'issue de l'étape b) montre un aspect homogène et limpide, de viscosité 11,000 mPa.s (mesurée à l'aide d'un viscosimètre de type Brookfield LVT, à une vitesse de 6 tours/minute). Après une période de stockage de 6 mois à 25°C, le gel obtenu à l'issue de l'étape b) de ce mode opératoire présente un aspect homogène et limpide, de viscosité 12,000 mPa.s (mesurée à l'aide d'un viscosimètre de type Brookfield LVT, à une vitesse de 6 tours/minute).

**2.3.2 Procédé de nettoyage mettant en oeuvre la composition préparée en 2.3.1 :**

La compositon préparée en 2.3.1, se présentant sous la forme d'un gel, est pulvérisée à température ambiante sur les parois d'un four souillé par des graisses alimentaires et sur les grilles de cuissons également souillées par des graisses alimentaires. Après une durée de 10 minutes, les parois du fours et les grilles de cuisson sont rincées à l'aide d'eau chaude à 60°C. Les parois du four et la surfaces des grilles de cuisson ainsi nettoyé ne présentent plus de salissures.

**Revendications**

**1.**  Composition de formule (I) :

$$nC_7H_{15}\text{-}O\text{-}(G)_p\text{-}H \qquad (I)$$

dans laquelle G représente le reste d'un sucre réducteur et p représente un nombre décimal supérieur ou égal à 1,05 et inférieur ou égal à 5, ladite formule (I) représentant un mélange de composés :

$$a_1\ nC_7H_{15}\text{-}O\text{-}(G)_1\text{-}H + a_2\ nC_7H_{15}\text{-}O\text{-}(G)_2\text{-}H + a_3\ nC_7H_{15}\text{-}O\text{-}(G)_3\text{-}H + ... + a_q\ nC_7H_{15}\text{-}O\text{-}(G)_q\text{-}H$$

avec q représentant un nombre entier compris entre 1 et 5 et dans les proportions molaires $a_1$, $a_2$, $a_3$,... $a_q$ telles que :

$$\sum_{q=5}^{q=1} a_q = 1 \; ; \; a_1 > 0.$$

**2.** Composition de formule (I) telle que définie à la revendication 1, dans laquelle le reste G d'un sucre réducteur est choisi parmi les restes du glucose, du xylose et de l'arabinose et p représente un nombre décimal supérieur ou égal à 1,05 et inférieur ou égal à 2,5.

**3.** Procédé de préparation de la composition de formule (I) telle que définie à l'une quelconque des revendications 1 ou 2, comprenant les étapes successives suivantes :

une étape A) de réaction d'un sucre réducteur de formule (III) :

$$HO\text{-}(G)_p\text{-}H \qquad (III)$$

dans laquelle G représente le reste d'un sucre réducteur,
avec un excès de n-heptanol molaire de formule $C_7H_{15}$-OH, pour former un mélange de composés de formule (I) et de n-heptanol ;
une étape B) d'élimination du n-heptanol dudit mélange obtenu à l'étape A).

**4.** Utilisation de la composition de formule (I) telle que définie à l'une quelconque des revendications 1 ou 2, comme agent tensioactif hydrotrope.

**5.** Utilisation de la composition de formule (I) telle que définie à l'une quelconque des revendications 1 ou 2, comme agent solubilisant, dans une composition alcaline aqueuse, d'au moins un tensioactif non-ionique de formule (II) :

$$R\text{-}(O\text{-}CH(R')\text{-}CH_2)_n\text{-}(O\text{-}CH_2\text{-}CH_2)_m\text{-}O\text{-}H \qquad (II)$$

dans laquelle R représente un radical aliphatique hydrocarboné, saturé ou insaturé, linéaire ou ramifié, comportant de 8 à 14 atomes de carbone, R' représente un radical méthyl ou éthyl, n représente un nombre entier supérieur ou égal à 0 et inférieur ou égal à 15, m représente un nombre entier supérieur ou égal à 0 et inférieur ou égal à 15, étant entendu que la somme n + m est supérieure à zéro.

**6.** Utilisation telle que définie à la revendication 5, **caractérisée en ce que** dans la formule (II), le radical R représente un radical choisi parmi les radicaux octyle, décyle, dodécyle, tétradécyle, 2-éthyl hexyle, 2-butyl octyle, 2-butyl décyle, 2-hexyl octyle, isooctyle, isononyle, isodécyle, isoundécyle, isododécyle, isotridécyle, isotétradécyle ou 2-propyl heptyle.

**7.** Utilisation telle que définie à l'une quelconque des revendications 5 ou 6, **caractérisée en ce que** dans la formule (II), n représente un nombre entier supérieur ou égal 0 et inférieur ou égal à 6, plus particulièrement supérieur ou égal à 0 et inférieur ou égal à 3, et encore plus particulièrement supérieur ou égal à 0 et inférieur ou égal à 2.

**8.** Utilisation telle que définie à l'une quelconque des revendications 5 à 7 **caractérisée en ce que** dans la formule (II), m représente un nombre entier supérieur ou égal à 1 et inférieur ou égal à 9, plus particulièrement supérieur ou égal à 2 et inférieur ou égal à 6, et encore plus particulièrement supérieur ou égal à 2 et inférieur ou égal à 4.

**9.** Utilisation telle que définie à l'une quelconque des revendications 5 à 8, **caractérisée en ce que** le rapport massique entre le composé de formule (II) et le composé de formule (I) est inférieur ou égal à 9/1 et supérieur ou égal à 1/4.

**10.** Composition $(C_1)$ comprenant pour 100% de sa masse :

a) de 0,5 % à 20 % massique de la composition de formule (I), telle que définie à la revendication1 ;
b) de 0,5 % à 80 % massique d'au moins un tensioactif non-ionique de formule (II) :

$$R\text{-}(O\text{-}CH(R')\text{-}CH_2)_n\text{-}(O\text{-}CH_2\text{-}CH_2)_m\text{-}O\text{-}H \qquad (II)$$

dans laquelle R représente un radical aliphatique hydrocarboné, saturé ou insaturé, linéaire ou ramifié, comportant de 8 à 14 atomes de carbone, R' représente un radical méthyle ou propyle, n représente un nombre entier supérieur ou égal à 0 et inférieur ou égal à 15 , m représente un nombre entier supérieur ou égal à 0 et inférieur ou égal à 15, étant entendu que la somme n + m est supérieure à zéro ;

c) de 10 % à 50 % massique d'au moins un agent alcalin choisi parmi les éléments du groupe constitué par les hydroxydes de métaux alcalins ou alcalino-terreux ;

d) de 15 % à 89 % massique d'eau ; et optionnellement

e) de 10 % à 50 % massique d'au moins un agent anticalcaire

**11.** Composition ($C_1$) telle que définie à la revendication 10, **caractérisée en ce que** le rapport massique entre le composé de formule (II) et la composition de formule (I) est inférieur ou égal à 9/1 et supérieur ou égal à 1/4.

**12.** Utilisation de la composition ($C_1$) telle que définie à l'une quelconque des revendications 10 ou 11, pour nettoyer les surfaces dures.

**13.** Procédé de nettoyage d'une surface dure, **caractérisé en ce qu'**il comprend :

au moins une étape $a_1$) d'application de la composition ($C_1$) telle que définie à l'une des revendications 10 ou 11 sur ladite surface dure, suivie

d'au moins une étape $b_1$) de rinçage de ladite surface dure.

## Patentansprüche

**1.** Verbindung mit der Formel (I):

$$nC_7H_{15}\text{-}O\text{-}(G)_p\text{-}H \qquad (I)$$

worin G für den Rest eines reduzierenden Zuckers steht und p für eine Dezimalzahl größer oder gleich 1,05 und kleiner oder gleich 5 steht, wobei die Formel (I) für ein Gemisch aus Verbindungen steht:

$$a_1 nC_7H_{15}\text{-}O\text{-}(G)_1\text{-}H + a_2 nC_7H_{15}\text{-}O\text{-}(G)_2\text{-}H + a_3 nC_7H_{15}\text{-}O\text{-}(G)_3\text{-}H + ... a_q nC_7H_{15}\text{-}O\text{-}(G)_q\text{-}H$$

wobei q für eine ganze Zahl im Bereich zwischen 1 und 5 steht und in Molanteilen $a_1$, $a_2$, $a_3$,... aq, so dass:

$$\sum_{q=1}^{q=1} a_q = 1 \; ; a_1 > 0.$$

**2.** Zusammensetzung mit der Formel (I) nach Anspruch 1, worin der Rest G eines reduzierenden Zuckers ausgewählt ist aus den Glucose-, Xylose- und Arabinoseresten und p für eine Dezimalzahl größer oder gleich 1,05 und kleiner oder gleich 2,5 steht.

**3.** Verfahren zur Herstellung der Zusammensetzung mit der Formel (I) nach einem der Ansprüche 1 oder 2, das die folgenden aufeinanderfolgenden Schritte umfasst:

einen Schritt A) zum Umsetzen eines reduzierenden Zuckers mit der Formel (III):

$$HO\text{-}(G)_p\text{-}H \qquad (III)$$

worin G für den Rest eines reduzierenden Zuckers steht,

mit einem Molüberschuss n-Heptanol mit der Formel $C_7H_{15}$-OH, um ein Gemisch von Verbindungen mit der Formel (I) und n-Heptanol zu bilden;

einen Schritt B) zum Entfernen des n-Heptanols aus dem in Schritt A) erhaltenen Gemisch.

**4.** Verwendung der Zusammensetzung mit der Formel (I) nach einem der Ansprüche 1 oder 2 als hydrotropes Tensid.

5. Verwendung der Zusammensetzung mit der Formel (I) nach einem der Ansprüche 1 oder 2 als Lösungsvermittler in einer wässrigen, alkalischen Zusammensetzung für mindestens ein nicht-ionisches Tensid mit der Formel (II):

$$R\text{-}(O\text{-}CH(R')\text{-}CH_2)_n\text{-}(O\text{-}CH_2\text{-}CH_2)_m\text{-}O\text{-}H \qquad (II)$$

worin R für ein gesättigtes oder ungesättigtes, lineares oder verzweigtes, aliphatisches Kohlenwasserstoffradikal steht, das 8 bis 14 Kohlenstoffatome umfasst, R' für ein Methyl- oder Ethylradikal steht, $n$ für eine ganze Zahl größer oder gleich 0 und kleiner oder gleich 15 steht, $m$ für eine ganze Zahl größer oder gleich 0 und kleiner oder gleich 15 steht, wobei gilt, dass die Summe von $n + m$ größer als null ist.

6. Verwendung nach Anspruch 5, **dadurch gekennzeichnet, dass** in der Formel (II) das Radikal R für ein Radikal steht, ausgewählt aus den Octyl-, Decyl-, Dodecyl-, Tetradecyl-, 2-Ethylhexyl-, 2-Butyl-octyl-, 2-Butyldecyl-, 2-Hexyloctyl-, Isooctyl-, Isononyl-, Isodecyl-, Isoundecyl-, Isododecyl-, Isotridecyl-, Isotetradecyl- oder 2-Propylheptylradikalen.

7. Verwendung nach einem der Ansprüche 5 oder 6, **dadurch gekennzeichnet, dass** in der Formel (II) $n$ für eine ganze Zahl größer oder gleich 0 und kleiner oder gleich 6, insbesondere größer oder gleich 0 und kleiner oder gleich 3 und noch stärker bevorzugt größer oder gleich 0 und kleiner oder gleich 2 steht.

8. Verwendung nach einem der Ansprüche 5 bis 7, **dadurch gekennzeichnet, dass** in der Formel (II) $m$ für eine ganze Zahl größer oder gleich 1 und kleiner oder gleich 9, insbesondere größer oder gleich 2 und kleiner oder gleich 6 und noch stärker bevorzugt größer oder gleich 2 und kleiner oder gleich 4 steht.

9. Verwendung nach einem der Ansprüche 5 bis 8, **dadurch gekennzeichnet, dass** das Masseverhältnis zwischen der Verbindung mit der Formel (II) und der Verbindung mit der Formel (I) kleiner oder gleich 9/1 und größer oder gleich 1/4 ist.

10. Zusammensetzung ($C_1$), umfassend für 100 % ihrer Masse:

     a) 0,5 Masse-% bis 20 Masse-% mindestens einer Verbindung mit der Formel (I) nach Anspruch 1;
     b) 0,5 Masse-% bis 80 Masse-% mindestens eines nichtionischen Tensids mit der Formel (II):

$$R\text{-}(O\text{-}CH(R')\text{-}CH_2)_n\text{-}(O\text{-}CH_2\text{-}CH_2)_m\text{-}O\text{-}H \qquad (II)$$

worin R für ein gesättigtes oder ungesättigtes, lineares oder verzweigtes, aliphatisches Kohlenwasserstoffradikal steht, das 8 bis 14 Kohlenstoffatome umfasst, R' für ein Methyl- oder Propylradikal steht, $n$ für eine ganze Zahl größer oder gleich 0 und kleiner oder gleich 15 steht, $m$ für eine ganze Zahl größer oder gleich 0 und kleiner oder gleich 15 steht, wobei gilt, dass die Summe von $n + m$ größer als null ist;
     c) 10 Masse-% bis 50 Masse-% mindestens eines alkalischen Mittels, ausgewählt aus den Elementen der Gruppe bestehend aus den Alkali- oder Erdalkalimetallhydroxiden;
     d) 15 Masse-% bis 89 Masse-% Wasser; und wahlweise
     e) 10 Masse-% bis 50 Masse-% mindestens eines Kalkschutzmittels.

11. Zusammensetzung ($C_1$) nach Anspruch 10, **dadurch gekennzeichnet, dass** das Masseverhältnis zwischen der Verbindung mit der Formel (II) und der Verbindung mit der Formel (I) kleiner oder gleich 9/1 und größer oder gleich 1/4 ist.

12. Verwendung der Zusammensetzung ($C_1$) nach einem der Ansprüche 10 oder 11 zum Reinigen von harten Oberflächen.

13. Verfahren zum Reinigen einer harten Oberfläche, **dadurch gekennzeichnet, dass** es umfasst:

     mindestens einen Schritt $a_1$) zum Auftragen der Zusammensetzung ($C_1$) nach einem der Ansprüche 10 oder 11 auf die harte Oberfläche, gefolgt von
     mindestens einem Schritt $b_1$) zum Spülen der harten Oberfläche.

**Claims**

1. Composition of formula (I):

$$nC_7H_{15}\text{-O-}(G)_p\text{-H} \qquad (I)$$

wherein G denotes the group of a reducing sugar and p denotes a decimal number greater than or equal to 1.05 and less than or equal to 5, said formula (I) representing a mixture of compounds:

$$a_1 nC_7H_{15}\text{-O-}(G)_1\text{-H} + a_2 nC_7H_{15}\text{-O-}(G)_z\text{-H} + a_3 nC_7H_{15}\text{-O-}(G)_3\text{-H} + ... + a_q\, nC_7H_{15}\text{-O-}(G)q\text{-H}$$

where q denotes an integer between 1 and 5 and in the molar proportions $a_1$, $a_2$, $a_3$,...$a_q$ such that:

$$\sum_{q=5}^{q=1} a_q = 1\,; a_1 > 0$$

2. Composition of formula (I) as defined in claim 1, wherein the group G of a reducing sugar is selected from among the glucose, xylose and arabinose groups and p denotes a decimal number greater than or equal to 1.05 and less than or equal to 2.5.

3. Method for preparing the composition of formula (I) as defined in any one of claims 1 or 2, comprising the following successive steps:

   a step A) of reacting a reducing sugar of formula (III):

   $$HO\text{-}(G)_p\text{-H} \qquad (III)$$

   wherein G denotes the group of a reducing sugar,
   with a molar excess of n-heptanol of formula $C_7H_{15}$-OH to form a mixture of compounds of formula (I) and n-heptanol;
   a step B of eliminating the n-heptanol from said mixture obtained in step A).

4. Use of the composition of formula (I) as defined in any one of claims 1 and 2, as a hydrotropic surfactant.

5. Use of the composition of formula (I) as defined in any one of claims 1 and 2, as a solubiliser, in an aqueous alkaline composition, for at least one non-ionic surfactant of formula (II):

   $$R\text{-}(O\text{-}CH(R')\text{-}CH_2)_n\text{-}(O\text{-}CH_2\text{-}CH_2)_m\text{-}O\text{-}H \qquad (II)$$

   wherein R denotes a straight-chain or branched, saturated or unsaturated, aliphatic hydrocarbon radical comprising 8 to 14 carbon atoms, R' denotes a methyl or ethyl radical, n denotes an integer greater than or equal to 0 and less than or equal to 15, m denotes an integer greater than or equal to 0 and less than or equal to 15, it being understood that the sum n + m is greater than zero.

6. Use as defined in claim 5, **characterised in that** in formula (II) the radical R denotes a radical selected from among the octyl, decyl, dodecyl, tetradecyl, 2-ethyl-hexyl, 2-butyl-octyl, 2-butyl-decyl, 2-hexyl-octyl, isooctyl, isononyl, isodecyl, isoundecyl, isododecyl, isotridecyl, isotetradecyl or 2-propyl-heptyl radicals.

7. Use as defined in any one of claims 5 or 6, **characterised in that** in formula (II) n denotes an integer greater than or equal to 0 and less than or equal to 6, more particularly greater than or equal to 0 and less than or equal to 3, and still more particularly greater than or equal to 0 and less than or equal to 2.

8. Use as defined in any one of claims 5 or 7, **characterised in that** in formula (II) m denotes an integer greater than or equal to 1 and less than or equal to 9, more particularly greater than or equal to 2 and less than or equal to 6, and still more particularly greater than or equal to 2 and less than or equal to 4.

9. Use as defined in any one of claims 5 to 8, **characterised in that** the mass ratio between the compound of formula (II) and the compound of formula (I) is less than or equal to 9/1 and greater than or equal to 1/4.

10. Composition ($C_1$) comprising per 100% of its mass:

   a) 0.5% to 20% by mass of the composition of formula (I) as defined in claim 1;
   b) 0.5% to 80% by mass of at least one non-ionic surfactant of formula (II):

   $$R\text{-}(O\text{-}CH(R')\text{-}CH_2)_n\text{-}(O\text{-}CH_2\text{-}CH_2)_m\text{-}O\text{-}H \qquad (II)$$

   wherein R denotes a straight-chain or branched, saturated or unsaturated, aliphatic hydrocarbon radical comprising 8 to 14 carbon atoms, R' denotes a methyl or propyl radical, n denotes an integer greater than or equal to 0 and less than or equal to 15, m denotes an integer greater than or equal to 0 and less than or equal to 15, it being understood that the sum $n + m$ is greater than zero;
   c) from 10% to 50% by mass of at least one alkaline agent selected from among the elements of the group consisting of the alkali metal or alkaline earth metal hydroxides;
   d) from 15 to 89% by mass of water; and optionally
   e) from 10% to 50% by mass of at least one anti-scaling agent.

11. Composition ($C_1$) as defined in claim 10, **characterised in that** the mass ratio between the compound of formula (II) and the composition of formula (I) is less than or equal to 9/1 and greater than or equal to 1/4.

12. Use of the composition ($C_1$) as defined in any one of claims 10 and 11 for cleaning hard surfaces.

13. Method for cleaning a hard surface, **characterised in that** it comprises:

   at least one step $a_1$ of applying the composition ($C_1$) as defined in one of claims 10 or 11 to said hard surface, followed by
   at least one step $b_1$ of rinsing said hard surface.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 9633255 A1

- WO 9921948 A1

**Littérature non-brevet citée dans la description**

- **DANIEL VOET ; JUDITH G. VOET.** Biochemistry. John Wyley & Sons, 1990, 250